# EUROPEAN PATENT APPLICATION

(11) **EP 4 474 482 A1**
(43) Date of publication of application: **11.12.2024**
(21) Application number: 23749901.7
(22) Date of filing: 02.02.2023
(51) Int. Cl.: C12Q 1/02, C12Q 1/6816, C12N 15/11

(54) **CELL LABELLING METHOD**

(30) Priority: 04.02.2022 JP 2022016576
(71) Applicant: RIKEN, Wako-shi, Saitama 351-0198 (JP)
(72) Inventor: SUGIMOTO, Michihiko, Wako-shi, Saitama 351-0198 (JP); ABE, Kuniya, Wako-shi, Saitama 351-0198 (JP)
(74) Representative: Mewburn Ellis LLP
(86) International application number: PCT/JP2023/004476
(87) International publication number: WO 2023/149585

(57) **Abstract**

The present invention relates to a method for labeling a cell with a barcode. The method for labeling a cell with a barcode of the invention includes (1) for a cell group containing a plurality of single cells, directly or indirectly bringing a cell surface protein of the cell into contact with a modified barcode; or (2) directly or indirectly bringing a cell surface protein of a single cell into contact with a modified barcode.

## Description

### TECHNICAL FIELD

The present invention relates to a method for labeling cells with barcodes, and a kit for use in the method. The present invention also relates to a method for multiplexed analysis of cell samples.

### BACKGROUND ART

Comprehensive gene expression analysis is an essential technology for analyzing properties of cells and tissues. Comparing gene expression profiles in different cells and tissues allows to reveal the diversity of cell types present, properties of various cells in different tissues, and their ratios in populations. In the conventional bulk analysis, numerous cells are put together to extract mRNAs from mixed samples and to be subjected to expression analysis, which means seeing the average of the gene expression in their cells. However, biological tissues are composed of complex and diverse cell populations. Therefore, it is difficult to grasp the cell conditions in the tissue and accurately understand the groups of expressed genes and their functions by the bulk analysis which uses the whole tissue. In addition, even cultured cell lines which are considered to be homogeneous can strictly be heterogeneous cell populations. Therefore, the bulk RNA-seq analysis is unable to accurately grasp the changes in gene expression that fluctuate according to, for example, the cell cycle or other modalities.

To overcome such a problem, the SINGLE CELL RNA-seq analysis (scRNA-seq analysis) is a very useful technique (NPL 1). The scRNA-seq includes capturing SINGLE CELLs ("SINGLE CELL" herein is used as a "cell group containing a plurality of single cells, or a single cell", unless otherwise specified), and creating an RNA-seq library from each cell for transcriptome analysis. Nevertheless, to use a large number of single cells as samples requires a huge amount of labor and cost, and the scRNA-seq was not an easily applicable technique until recently. However, techniques such as the droplet scRNA-seq in which a microfluidic/microdroplet reaction system is used, or an scRNA-seq in which a microwell is used have currently been developed, and equipment for these techniques has been developed and commercially available (e.g., BD Rhapsody system (NPL 2), and 10x chromium (NPL 3)). Progression of techniques in such a SINGLE CELL analysis has allowed to prepare a scRNA-seq library from 10,000 cells or more, leading to significantly reduce the analyzing cost per cell (NPL 4).

In spite of the fact that the cost per cell can be reduced, the analyzing cost as a whole is still high when analyzing a plurality of samples or performing time series analysis at a plurality of points of time. In addition, when performing the scRNA-seq experiment on a plurality of samples at different points of time and comparing them, analysis errors in each experiment batch (i.e., batch effects) may occur (NPL 5). Therefore, if a plurality of samples can be combined together and simultaneously processed in one library preparation, not only the analysis cost is significantly reduced, but also the batch effects can be lowered to perform a more precise scRNA-seq analysis. A technique for combining a plurality of samples together and analyzing them in a single experimental run is referred to as "sample multiplexing".

In sample multiplexing methods, a plurality of cell populations are labeled in advance with barcoded DNA tags having corresponding respective independent sequences, and then mixed together to perform SINGLE CELL analysis. From the RNA-seq data thus obtained, the origin of each cell can be traced based on the barcoded DNA sequences. The droplet scRNA-seq analysis or scRNA-seq analysis using microwells can cause a plurality of cells to be captured in one compartment, and such a multiple cell contamination error makes data interpretation difficult. Nevertheless, if different samples can be distinguished by different DNA tags, errors caused by multiplets can be reduced.

Regarding a multiplexing method using this principle, for example, a method of using an antibody for cell labeling bound to a barcoded oligo DNA as an antibody that recognizes a specific cell surface protein has been reported (NPL 6). However, an issue on this cell hashing method is on the assumption that the cell surface protein is expressed in all cells in the samples to be analyzed. Cell hashtag antibodies are antibody cocktails to be used for the purpose of comprehensively labeling cells originating from adult tissues (e.g., TotalSeq (R) anti-mouse hashtag antibody, BioLegend, Inc.). In a case of hashtags, antibodies to CD45 and MHC Class I are used (the former is for detecting cells of the immune system, and the latter is generally for detecting differentiated cells). However, for example, pluripotent stem cells, which are often used as an embryogenesis model, do not express CD45 or MHC Class I, and therefore the above method cannot be applicable (NPLs 7 and 8). Also, the above method has been found to be not applicable to some non-embryonic cells such as chondrocytes.

In addition to that, as a labeling technique for multiplexing, a transient barcoding method in which barcoded DNA is introduced into a cell (NPL 11), a CellTag indexing method in which lentivirus is used for introduction of barcoded DNA introduction (NPL 12), and the like have been developed. However, these methods are difficult to be applied to cells other than cultured cells, and therefore, the versatility is limited. Further, the ClickTag multiplexing method in which click chemistry and NHS-ester chemistry are combined (NPL 13) is a technique for attaching a barcoded DNA tag to a cell protein; however, the labeling efficiency of viable cells is poor, and this method can only be applied to methanol fixed cells. The CellPlex method (3' CellPlex Kit, 10x Genomics) uses a reagent that introduces a DNA tag into lipid in the cell membrane for universal cell labeling (NPL 14), but it is difficult to be applied to methanol fixed cells.

### CITATION LIST

### PATENT LITERATURE

PTL 1: WO 2020/010366 A1

### NON PATENT LITERATURE

NPL 1: X. Han, R. Wang, Y. Zhou, L. Fei, H. Sun, S. Lai, A. Saadatpour, Z. Zhou, H. Chen, F. Ye, D. Huang, Y. Xu, W. Huang, M. Jiang, X. Jiang, J. Mao, Y. Chen, C. Lu, J. Xie, Q. Fang, Y. Wang, R. Yue, T. Li, H. Huang, S.H. Orkin, G.-C. Yuan, M. Chen, G. Guo, Mapping the mouse cell atlas by Microwell-seq, Cell 172 (2018) 1091-1107, https://doi.org/10.1016/j.cell.2018.02.001.
[NPL 2] S.J. Yoon, L.S. Elahl, A.M. Pasca, R.M. Marton, A. Gordon, O. Revah, Y. Miura, E.M. Walczak, G.M. Holdgate, H.C. Fan, J.R. Huguenard, D.H. Geschwind, S.P. Pasca, Reliability of human cortical organoid generation. Nat. Methods 16 (2019) 75-78, https://doi.org/10.1038/s41592-018-0255-0.
[NPL 3] G.X.Y. Zheng, J.M. Terry, P. Belgrader, P. Ryvkin, Z.W. Bent, R. Wilson, S.B. Ziraldo, T.D. Wheeler, G.P. McDermott, J. Zhu, M.T. Gregory et. al. , Massively parallel digital transcriptional profiling of single cells, Nat. Commun. 8 (2017 Jan 16) 14049, https://doi.org/10.1038/ncomms14049.
[NPL 4] J. Cheng, J. Liao, X. Shao, X. Lu, X. Fan, Multiplexing methods for simultaneous large-scale transcriptomic profiling of samples at single-cell resolution, Adv. Sci. 8 (2021) 2101229, https://doi.org/10.1002/advs.202101229.
[NPL 5] H.T.N. Tran, K.S. Ang, M. Chevier, X. Zhang, N.Y.S. Lee, M. Goh, J. Chen, A benchmark of batch-effect correction methods for single-cell RNA sequencing data, Genome Biol. 21 (2020) 12, https://doi.org/10.1186/s13059-019-1850-9.
[NPL 6] M. Stoeckius, S. Zheng, B. Houck-Loomis, S. Hao, B.Z. Yeung, W.M. Mauck III, P. Smibert, R. Satija, Cell hashing with barcoded antibodies enables multiplexing and doublet detection for single cell genomics, Genome Biol. 19 (2018) 224, https://doi.org/10.1186/s13059-018-1603-1.
[NPL 7] S.L. McKinney-Freeman, O. Naveiras, F. Yates, S. Loewer, M. Philitas, M. Curran, P.J. Park, G.Q. Daley, Surface antigen phenotypes of hematopoietic stem cells from embryos and murine embryonic stem cells, Blood 114 (2009) 268-278, https://doi.org/10.1182/blood-2008-12-193888.
[NPL 8] A.S. Boyd, K.J. Wood, Variation in MHC expression between undifferentiated mouse ES cells and ES cell-derived insulin-producing cell clusters, Transplantation 87 (2009) 1300-1304, https://doi.org/10.1097/TP.0b013e3181a19421.
[NPL 9] T. Redmer, S. Diecke, T. Grigoryan, A. Quiroga-Negreira, W. Birchmeier, D. Besser, E-cadherin is crucial for embryonic stem cell pluripotency and can replace OCT4 during somatic cell reprogramming, EMBO Rep. 12 (2011) 720-726, https://doi.org/10.1038/embor.2011.88.
[NPL 10] S. Shichino, S. Ueha, S. Hashimoto, T. Ogawa, H. Aoki, W. Bin, C.-Y. Chen, M. Kitabatake, N. Ouji-Sageshima, S. Hontsu, N. Sawabata, T. Kawaguchi, T. Okayama, E. Sugihara, T. Ito, K. Ikeo, T. Sato, K. Matsushima, TAS-Seq: a robust and sensitive amplification method for beads-based scRNA-seq, bioRxiv (2021), https://doi.org/10.1101/2021.08.03.454735.
[NPL 11] D. Shin, W. Lee, J.H. Lee, D. Bang, Multiplexed single-cell RNA-seq via transient barcoding for simultaneous expression profiling of various drug perturbations, Sci. Adv. 5 (2019) eaav2249, https://doi.org/10.1126/sciadv.aav2249.
[NPL 12] C. Guo, W. Kong, K. Kamimoto, G.C. Rivera-Gonzalez, X. Yang, Y. Kirita, S.A. Morris, CellTag Indexing: genetic barcode-based sample multiplexing for single-cell genomics. Genome Biol. 20 (2019) 90, https://doi.org/10.1186/s13059-019-1699-y.
[NPL 13] J. Gehring, J.H. Park, S. Chen, M. Thomson, L. Pachter, Highly multiplexed single-cell RNA-seq by DNA oligonucleotide tagging of cellular proteins, Nat. Biotechnol. 38 (2020) 35-38, https://doi.org/10.1038/s41587-019-0372-z.
[NPL 14] C. McGinnis, D.M. Patterson, J. Winkler, D.N. Conrad, M.Y. Hein, V. Srivastava, J.L. Hu, L.M. Murrow, J.S. Weissman, Z. Werb, E.D. Chow, Z.J. Gartner, MULTI-seq: sample multiplexing for single-cell RNA sequencing using lipid-tagged indices, Nat. Methods 16 (2019) 619-626, https://doi.org/10.1038/s41592-019-0433-8.
[NPL 15] H. Niwa, S. Masui, I. Chambers, A.G. Smith, J. Miyazaki, Phenotypic complementation establishes requirements for specific POU domain and generic transactivation function of Oct-3/4 in embryonic stem cells, Mol. Cell. Biol. 22 (2002) 1526-1536, https://doi.org/10.1128/MCB.22.5.1526-1536.2002.
[NPL 16] K. Ogawa, H. Matsui, S. Ohtsuka, H. Niwa, A novel mechanism for regulating clonal propagation of mouse ES cells, Genes Cells 9 (2004) 471-477, https://doi.org/10.1111/j.1356-9597.2004.00736.x.
[NPL 17] M. Sugimoto, M. Kondo, M. Hirose, M. Suzuki, K. Mekada, T. Abe, H. Kiyonari, A. Ogura, N. Takagi, K. Artzt, K. Abe, Molecular identification of tw5: Vps52 promotes pluripotential cell differentiation through cell-cell interactions, Cell Rep. 2 (2012) 1363-1374, https://doi.org/10.1016/j.celrep.2012.10.004.
[NPL 18] Package 'Seurat' January 14, 2022 Version 4.1.0, Title Tools for Single Cell Genomics, http://ftp 1.us.debian.org/pub/cran/web/packages/Seurat/Seurat.pdf

### SUMMARY OF INVENTION

### TECHNICAL PROBLEM

The present inventors have made intensive studies to solve the above problems, and as a result, have developed a novel cell labeling technique for multiplexing scRNA-seq analysis that does not depend on specific cell surface antigens, and therefore have conceived the present invention. The method of the present invention has characteristics, one of which is including a step of directly or indirectly bringing a cell surface protein of a cell into contact with a modified barcode. In one aspect, the present invention is a universal surface biotinylation (USB) method. In another aspect, the method of the present invention is a method for directly binding a barcode to a cell surface protein of a cell (single-step method).

### SOLUTION TO PROBLEM

The present invention includes, but not limited to, the following aspects.
[1] A method for labeling a cell with a barcode, including:
   (1) for a cell group containing a plurality of single cells, directly or indirectly bringing a cell surface protein of the cell into contact with a modified barcode; or
   (2) directly or indirectly bringing a cell surface protein of a single cell into contact with a modified barcode.
[2] A method for labeling a cell with a barcode, including:
   (ia) for a cell group containing a plurality of single cells, biotinylating cell surface proteins of the cell;
   (ib) biotinylating cell surface proteins of a single cell; or
   (ic) biotinylating cell surface proteins of a plurality of cells followed by separating the biotinylated cells into cell groups containing a plurality of single cells, or into a single cell,
      and
   (ii) bringing each cell group or each single cell into contact with a barcoded biotin binding substance.
[3] The method according to [2], wherein the method including: (ia) for the cell group containing a plurality of single cells, biotinylating the cell surface protein;
   and
   (ii) bringing each cell group into contact with the barcoded biotin binding substance.
[4] The method according to [2] or [3], including biotinylating an amino group, a sulfhydryl group, or a carboxyl group of the cell surface protein in (i).
[5] The method according to any one of [2] to [4], in which a reagent used for biotinylation in (i) is selected from the group consisting of sulfo-N-hydroxysuccinimide-biotin, N-hydroxysuccinimide-biotin, and pentafluorophenyl-biotin,
   in which the binding of biotin with sulfo-N-hydroxysuccinimide, N-hydroxysuccinimide, or pentafluorophenyl can include a spacer.
[6] The method according to [5], in which the reagent used for biotinylation in (i) has been cryopreserved.
[7] The method according to any one of [2] to [6], in which the biotin binding substance is selected from the group consisting of streptavidin, avidin, and anti-biotin antibody.
[8] The method according to [7], in which the biotin binding substance is streptavidin.
[9] The method according to any one of [1] to [8], in which the barcode is oligo DNA.
[10] The method according to [1] to [9], wherein the method is for labeling a viable cell.
[11] The method according to any one of [1] to [9], wherein the method is for labeling a fixed cell.
[12] A kit for use in a method for labeling a cell with a barcode, including a reagent for biotinylating a cell surface protein of the cell, and
   a barcoded biotin binding substance.
[13] The kit according to [12], in which the reagent for biotinylating the cell surface protein of the cell is sulfo-N-hydroxysuccinimide-biotin.
[14] A method for multiplexed analysis of a cell sample, including:
   (ia) for a cell group containing a plurality of single cells, biotinylating cell surface proteins of the cell;
   (ib) biotinylating cell surface proteins of a single cell; or
   (ic) biotinylating cell surface proteins of a plurality of cells followed by separating the biotinylated cells into cell groups containing a plurality of single cells, or into a single cell,
   (ii) bringing each cell group or each single cell into contact with a barcoded biotin binding substance,
   (iii) mixing all or a part of the cell groups or single cells barcode-labeled in (ii), and
   (iv) analyzing the mixture of the cells of (iii).
[15] The method according to [14], including: (ia) for the cell group containing a plurality of single cells, biotinylating the cell surface protein;
   and
   (ii) bringing each cell group into contact with the barcoded biotin binding substance.
[16] The method according to [15], in which the analysis is cellular RNA analysis.
[17] A method for labeling a cell with a barcode, including:
   (1) bringing a cell group containing a plurality of single cells into contact with a barcode modified with a compound that binds to an amino group of a cell surface protein of the cell; or
   (2) bringing a single cell into contact with a barcode modified with a compound that binds to an amino group of the cell surface protein of the cell.
[18] The method according to [17], in which the compound that binds to the amino group of the cell surface protein of the cell is selected from the group consisting of sulfo-N-hydroxysuccinimide, N-hydroxysuccinimide, and pentafluorophenyl.
[19] The method according to [18], in which the compound that binds to the amino group of the cell surface protein of the cell has been cryopreserved.
[20] The method according to any one of [17] to [19], in which the barcode is oligo DNA.
[21] The method according to [17] to [20], wherein the method is for labeling a viable cell.
[22] The method according to any one of [17] to [21], wherein the method is for labeling a fixed cell.
[23] A kit for use in a method for labeling a cell with a barcode, including a barcode modified with a compound that binds to an amino group of a cell surface protein of the cell.
[24] The kit according to [23], in which the compound that binds to the amino group of the cell surface protein of the cell is sulfo-N-hydroxysuccinimide.
[25] A method for multiplexed analysis of a cell sample, including:
   (1A) bringing a cell group containing a plurality of single cells into contact with a barcode modified with a compound that binds to an amino group of a cell surface protein of the cell; or
   (1B) bringing a single cell into contact with a barcode modified with a compound that binds to an amino group of a cell surface protein of the cell,
   (2) mixing all or a part of the cell groups or single cells barcode-labeled in (1), and
   (3) analyzing the mixture of the cells of (2).
[26] The method according to [25], in which the analysis is cellular RNA analysis.

### ADVANTAGEOUS EFFECTS OF INVENTION

Since the method for labeling a cell with a barcode of the present invention targets a cell surface protein of the cell in both the USB method and the single-step method, it is possible to label cells in any state as long as the cell surface protein exists. For example, it does not depend on the expression of cadherin 1 (CDH1), and therefore both undifferentiated cells and differentiated cells can be targeted. In addition, the type of cells to be labeled is not limited, and the method can be applied to all cell types. Further, it can also be applied to cells fixed with methanol or the like.

In the USB method, biotinylation is not dependent on specific cell surface proteins and can be carried out on any cell surface proteins. The method of the present invention, which uses biotinylation on cell surface proteins, is a universal labeling technique capable of comprehensively labeling cells. The technique of the present invention (hereinafter, referred to as "USB method") is highly versatile and applicable to any type of cell.

The method of the present invention enables labeling of all cell types by using biotin that can be introduced into any cell surface protein. The USB method does not give any adverse effect on the growth or colony formation ability of the tested cells. Further, it has been revealed in an experiment using the differentiation process of mouse embryonic stem (ES) cells as a model that when sample multiplexing and applying the USB method to the SINGLE CELL analysis, various differentiated cells are successfully captured without adverse effects on detection ability for genes or transcriptome acquisition on each cell. The USB method can be used for many cell types, including human iPS-derived chondrocytes and rat lung-derived cells, to which conventional cell hashing methods have not been successfully applied so far. The USB method can theoretically be applied to all cell types, and is convenient, highly efficient and cost effective method for multiplexing scRNA-seq.

The single-ste+p method includes a step of bringing into contact with a barcode modified with a compound that binds to an amino group of a cell surface protein of the cell. Since the single-step method also targets a cell surface protein of the cell as in the USB method, it is possible to label all cell types. The single-step method does not give any adverse effect on the growth or colony formation ability of the tested cells. Further, it has been confirmed in Examples herein that when applying the single-step method to the SINGLE CELL analysis, various differentiated cells are successfully captured without adverse effects on detection ability for genes or transcriptome acquisition on each cell.

The present invention can be used for disease analysis, such as performing scRNA-seq with cancer tissues. The present invention can also be used for confirming whether iPS cells have been differentiated into cell types of interest, in transplantation therapies with iPS cells used. The present invention can further be used to verify the effects of agents on patient-derived iPS cells, when screening candidates of therapeutic drugs for genetic diseases.

### BRIEF DESCRIPTION OF DRAWINGS

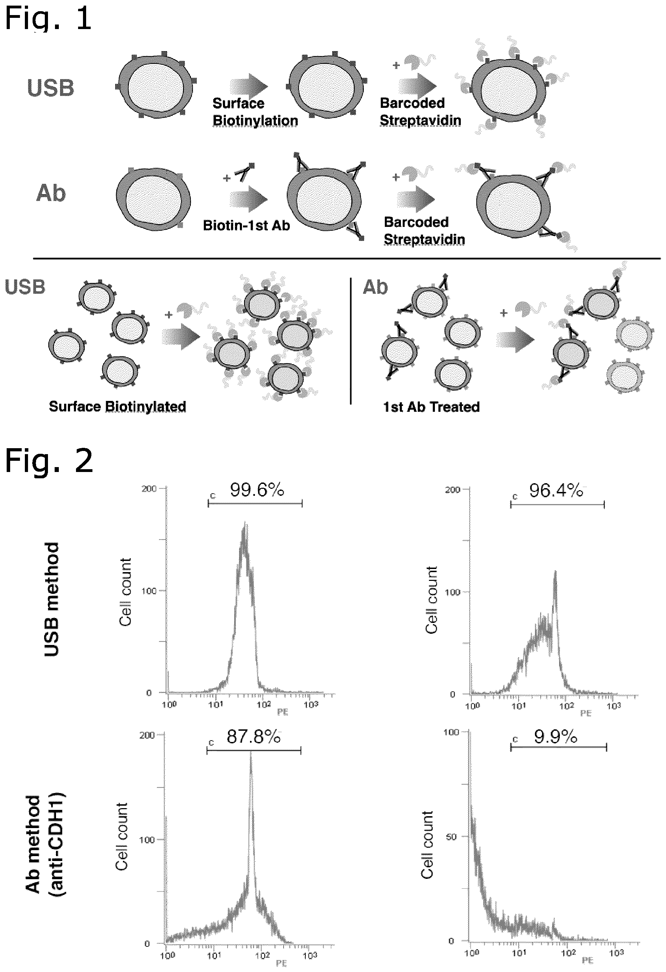

[Fig. 1] Fig. 1 illustrates the principle of the USB method and antibody method (Ab method). The USB method universally biotinylates cell surface proteins (blue) with S-NHS-biotin (biotinylated part in reddish brown color), and treats them with barcoded DNA-labeled streptavidin. The Ab method treats cells with biotin-conjugated antibodies against specific cell surface proteins (red), and then treats them with barcoded DNA-labeled streptavidin. When certain proteins are not expressed on some cells, these cells are not labeled by the Ab method, but all cells can be labeled by the USB method.
[Fig. 2] Fig. 2 shows flow cytometry results of undifferentiated ES cells and differentiated cells both labeled by the USB method and the Ab method. The ordinate indicates the cell count, and the abscissa indicates the fluorescence intensity of phycoerythrin (PE) in Fig. 2. The flow cytometry results revealed that in the USB method, the cell labeling efficiency is very high, with 99.6% of undifferentiated cells (upper left) and 96.4% of differentiated cells (upper right) being PE positive. In contrast, when using the Ab method, 87.8% of undifferentiated cells (lower left) and 9.9% of differentiated cells (lower right) were PE positive.

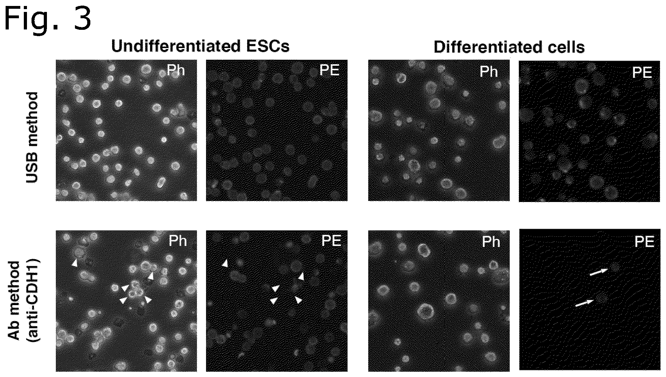

[Fig. 3] Fig. 3 is fluorescence photomicrographs of cells labeled by the USB method (upper panels) and the Ab method (lower panels) (magnification of x200). In the Ab method, an antibody against mouse CDH1 was used. CDH1 is known to be expressed on undifferentiated ES cells, and the expression was limited to cells in the undifferentiated state. (Upper) the USB method, (lower) the Ab method. (Left) undifferentiated ES cells, (right) differentiated cells (12 days after differentiation induction). Ph: phase-contrast images; PE: fluorescence images of TotalSeq(R)-PE-streptavidin; arrowheads in the left images: PE-negative cells; arrows in the right image: PE-positive cells.

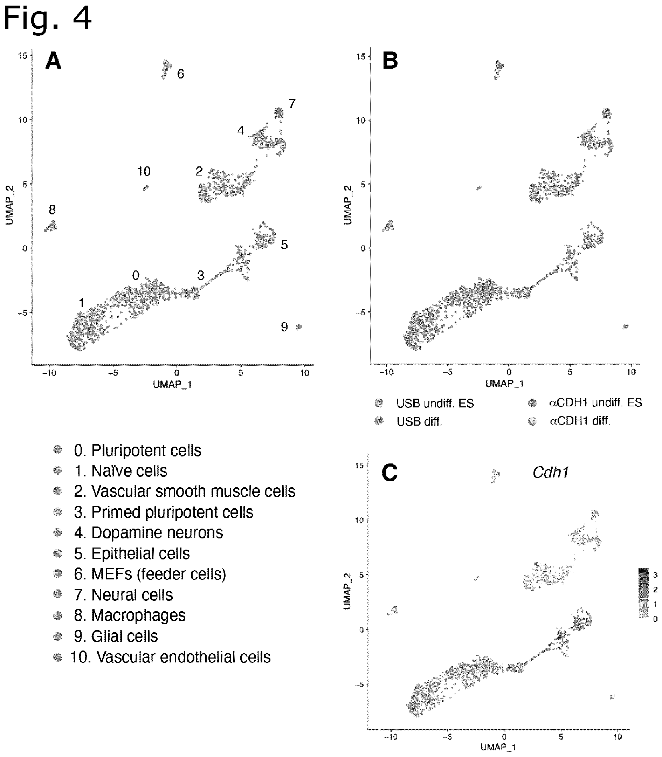

[Fig. 4] Fig. 4 shows results of scRNA-seq analysis of ES cells and differentiated derivatives thereof. For cell labeling, the USB multiplexing method or the Ab multiplexing method with CDH1 antibody was used. (A) By uniform manifold approximation and projection (UMAP) analysis, the cells were classified into 11 clusters: 0: pluripotent cells; 1: naive cells; 2: vascular smooth muscle cells; 3: pluripotent cells; 4: dopamine neurons; 5: epithelial cells; 6: MEFs (feeder cells); 7: neural cells; 8: macrophages; 9: glial cells; 10: vascular endothelial cells. (B) Distribution of cell samples on UMAP is shown. Color codes for each sample are shown below the figure. (C) Distribution of Cdh1-positive cells on UMAP is shown.

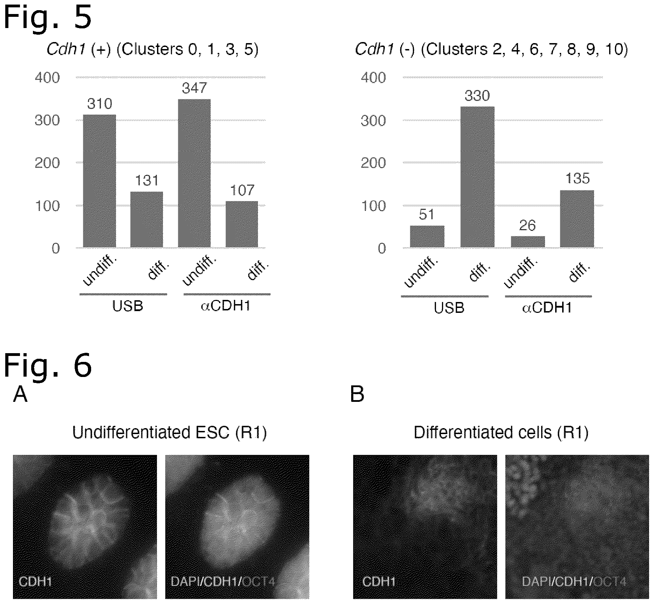

[Fig. 5] Fig. 5 is graphs showing the comparison of the cell counts detected in the USB method or the Ab method, in the clusters of Cdh1 positive (left) and Cdh1 negative (right) in Fig. 4.
[Fig. 6] Fig. 6 is results of immunostaining of CDH1 for (A) undifferentiated ES cells and (B) differentiated cells. CDH1 signals were observed on all of the cell surfaces in the undifferentiated ES cells, but detected on only a few cells in the differentiated cells. Green, CDH1; red, OCT4; blue, DAPI (nuclei).

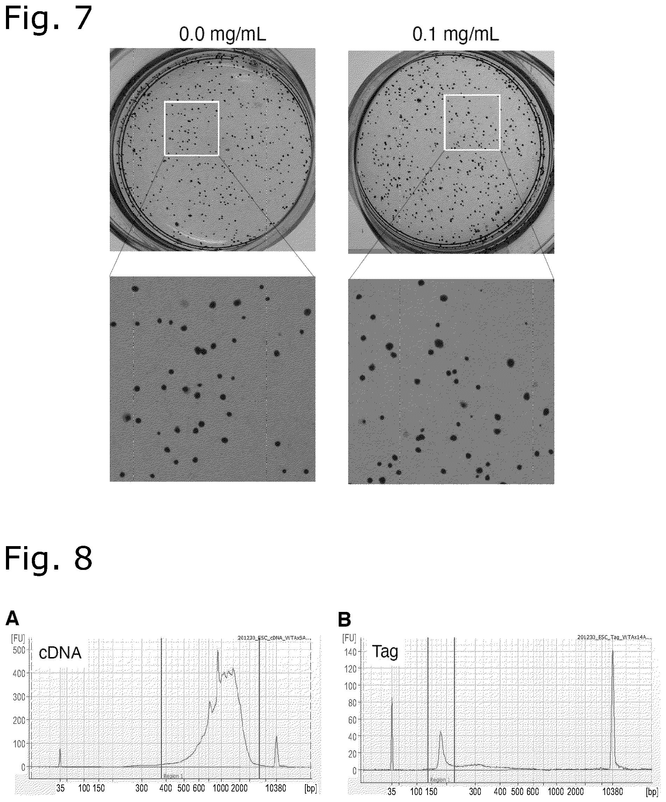

[Fig. 7] Fig. 7 is results by examining that S-NHS-biotin treatment does not affect the viability of the undifferentiated ES cells. (Right) cells treated with S-NHS-biotin and (left) control untreated cells. (Lower row) enlarged view x7.3.
[Fig. 8] Fig. 8 is graphs showing the quality check by electrophoresis for (A) all transcriptome cDNA amplified by TAS-seq protocol, and (B) barcoded DNA amplified by TAS-seq protocol. According to the electropherograms, the yields and size distributions of cDNA and barcoded DNA were sufficient for subsequent analysis.

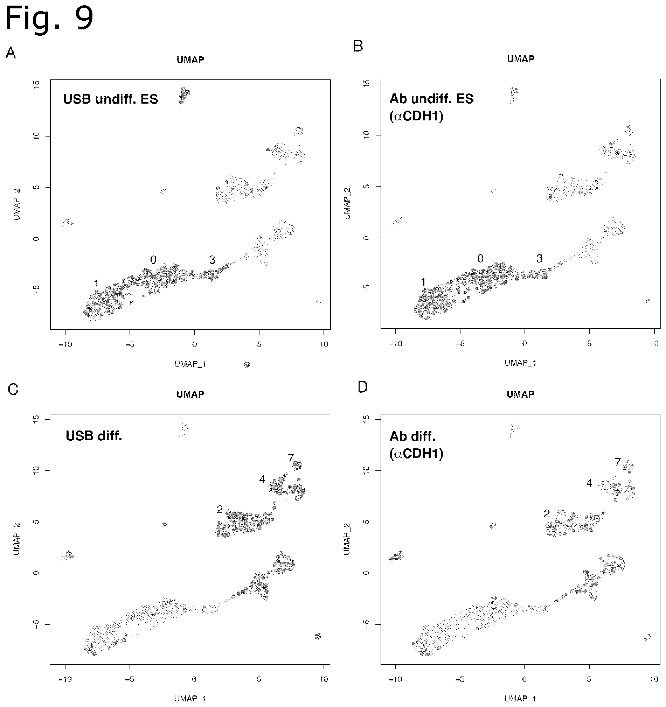

[Fig. 9] Fig. 9 shows results by examining, by the UMAP method, the distributions of the undifferentiated ES cells and the differentiated cells both labeled by the USB method and the Ab method. (A) UMAP distribution of the USB method-labeled undifferentiated ES cells; (B) UMAP distribution of the Ab method-labeled undifferentiated ES cells; (C) UMAP distribution of the USB method-labeled differentiated cells; and (D) UMAP distribution of the Ab method-labeled differentiated cells.

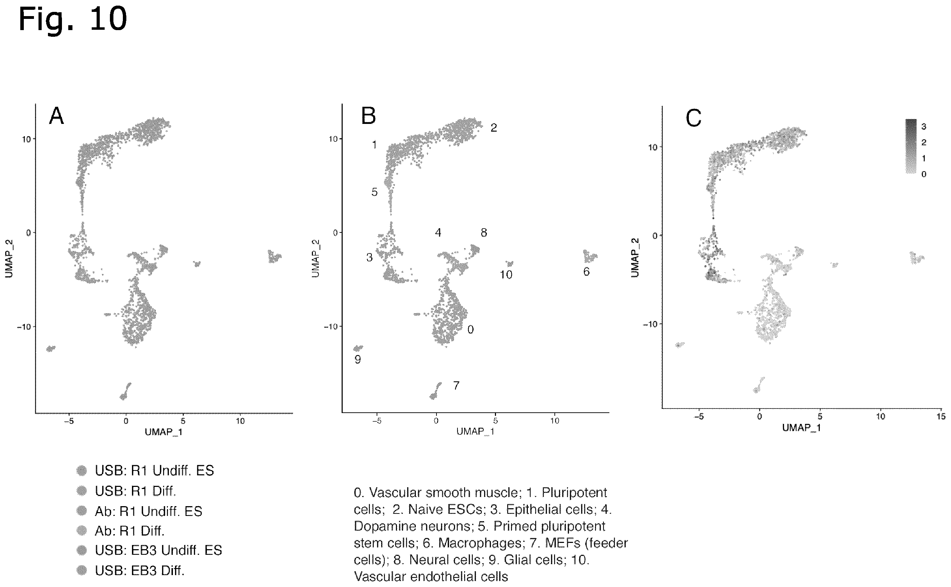

[Fig. 10] Fig. 10 shows results of scRNA-seq analysis of multiplexed samples. Used as the samples were a total of 6 samples of 4 samples of undifferentiated cells and differentiated cells derived from R1 ES cells labeled by either the USB method or the Ab method, and 2 samples of undifferentiated cells and differentiated cells derived from EB3 ES cell lines labeled by the USB method. The cell clusters were analyzed by the UMAP method, and the distribution of each sample is shown in (A). (B) 11 clusters were identified as follows. 0: vascular smooth muscle; 1: pluripotent cells; 2: naive ES cells; 3: epithelial cells; 4: dopamine neurons; 5: pluripotent stem cells; 6: macrophages; 7: MEFs (feeder cells); 8: neural cells; 9: glial cells; 10: vascular endothelial cells. (C) is results obtained by superimposing the Cdh1 expression level on the UMAP distribution in (A).

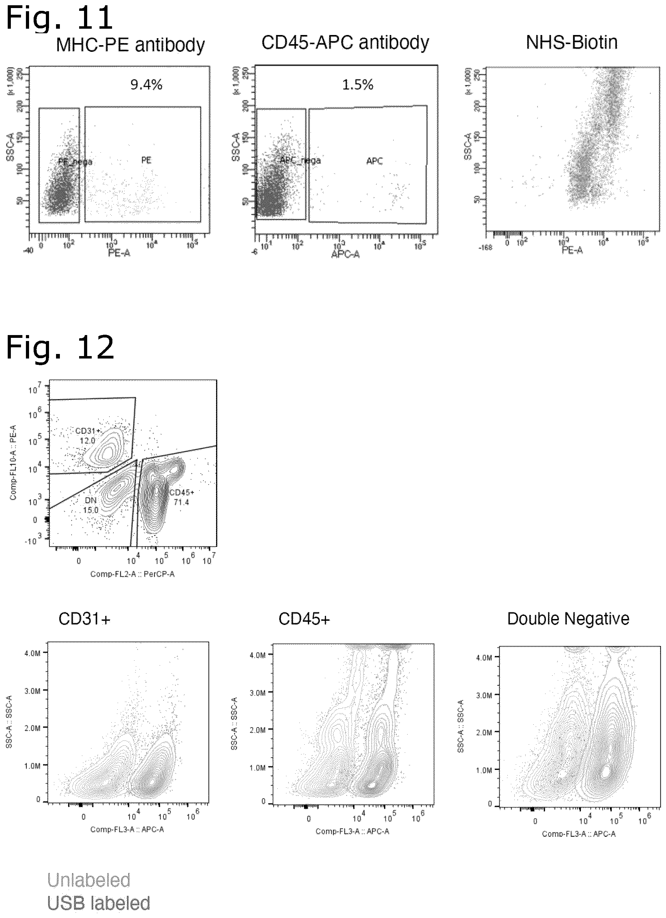

[Fig. 11] Fig. 11 is results of flow cytometry analysis after labeling, by the USB method, chondrocytes that cannot be labeled by the Ab method using existing reagents. The ordinate indicates side scattered light signals (SSC), and the abscissa indicates the fluorescence intensity of phycoerythrin (PE) in Fig. 11. Chondrocytes derived from mouse growth plates were each labeled with an MHC Class I antibody (left), a CD45 antibody (middle), and the USB method (right), and then analyzed by flow cytometry. Mouse growth plate chondrocytes, which are little stained with MHC and CD45 antibodies, are efficiently labeled with the USB method.
[Fig. 12] Fig. 12 is, as well as Fig. 11, the results of flow cytometry analysis after labeling, by the USB method, rat cells that cannot be labeled by the Ab method using existing reagents. The upper row of Fig. 12 shows results of flow cytometry analysis of rat lung cells double stained with PE-labeled CD 31 antibody and PerCP-labeled CD 45 antibody, whereas the lower row of Fig. 12 shows results of flow cytometry analysis by verifying the cell labeling efficiency by the USB method, using CD31 positive cells, CD45 positive cells and double negative cells. For experiments to verify the cell labeling efficiency by the USB method, APC-labeled streptavidin was used. The ordinate in the upper row of Fig. 12 indicates the fluorescence intensity of PE, and the abscissa indicates the fluorescence intensity of PerCP; and the ordinate in the lower row of Fig. 12 indicates side scattered light signals (SSC), and the abscissa indicates the fluorescence intensity of APC. Lung cells dissociated from rat lungs were fractionated into 3 populations of CD31 positive, CD45 positive and double negative. All fractionated cells were found to have been labeled by the USB method. The rats are of species for which a sample multiplexing kit based on the Ab method is not commercially available.

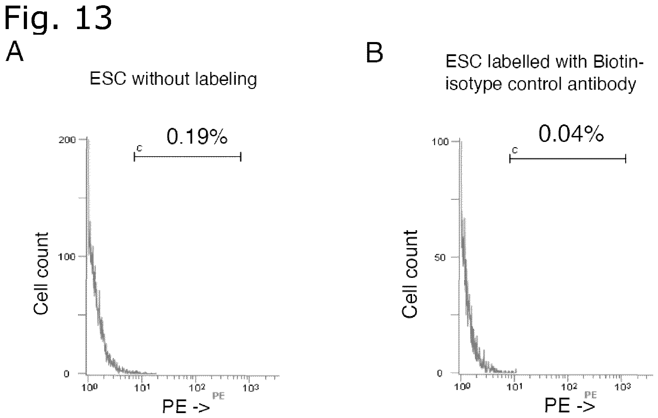

[Fig. 13] Fig. 13 shows verification results of presence or absence of non-specific binding of antibodies by flow cytometry analysis. The ordinate indicates the cell count, and the abscissa indicates the fluorescence intensity of phycoerythrin (PE) in Fig. 13. (A) Undifferentiated ES cells without labeling; (B) undifferentiated ES cells treated with a biotin-isotype control antibody and TotalSeq (R)-streptavidin (PE-conjugated). Non-specific binding of an isotype control antibody was not detected by flow cytometry analysis.

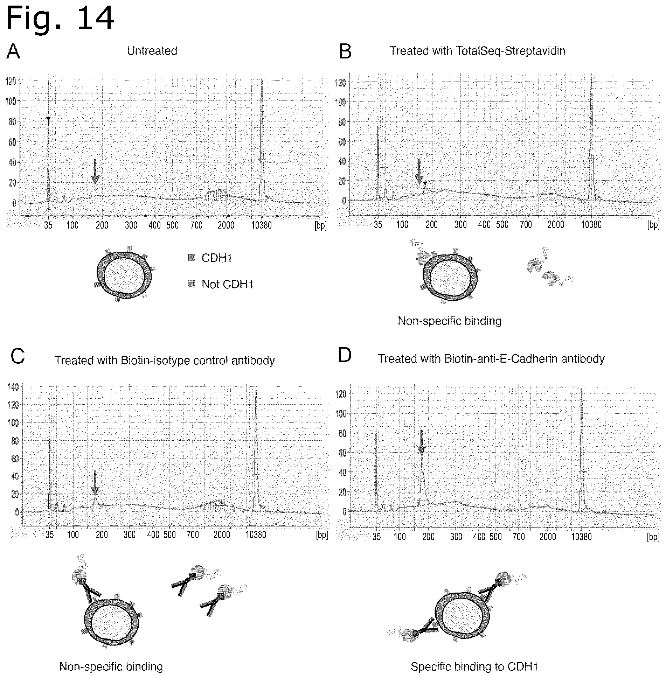

[Fig. 14] Fig. 14 shows non-specific binding of an isotype control antibody and barcode-tagged streptavidin (TotalSeq (R)-streptavidin) detected by the Tas-seq method. For each sample, cDNA synthesis reaction was performed, and barcoded DNA was amplified by the TAS-seq method. Next, the samples were analyzed using an Agilent bioanalyzer. (A) Cells untreated with an antibody and TotalSeq (R)-streptavidin (negative control sample); (B) cells treated with only TotalSeq (R)-streptavidin; (C) cells treated with an isotype control antibody followed by TotalSeq (R)-streptavidin; and (D) cells treated with a biotin-modified anti-CDH1 antibody followed by TotalSeq (R)-streptavidin (positive control sample). The relationship of sample reagents is schematically shown below each of the panels (A) to (D) (see also Fig. 1). The amplification of barcoded DNA was not observed in the negative control sample (A), whereas a clear peak (arrow) was observed in the positive control sample (D). Peaks (arrows) were observed in the other samples (B and C) despite smaller than that of (D), and the presence of non-specific binding of TotalSeq (R)-streptavidin and/or the isotype control antibody was observed. Using the TAS-seq method, which has high detection ability, resulted in observation of the non-specific binding of TotalSeq (R)-streptavidin and/or the antibody at weak levels that are undetectable by flow cytometry analysis. For the purpose of sample multiplexing in scRNA-seq analysis, the non-specific binding of TotalSeq (R)-streptavidin and/or the antibody is not obstacle for the Ab method; however, it is showed that the USB method is superior to the Ab method in technical severity.

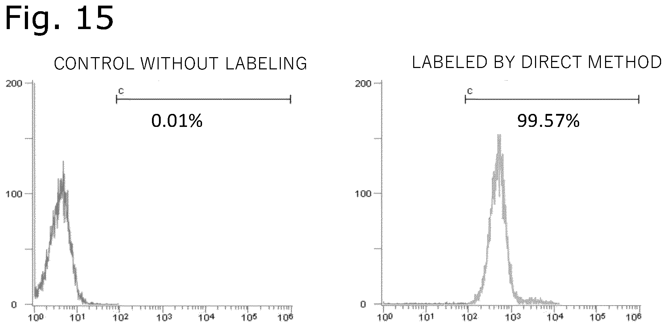

[Fig. 15] Fig. 15 shows results of labeling cells using sulfo-NHS-esterified oligo DNA (NEO) (single-step method) and verifying the labeling efficiency by flow cytometry analysis. To the 3'-end of NEO used for verification of the labeling efficiency in the single-step method, 6-FAM (6-carboxyfluorescein) was added. The ordinate indicates the cell count, and the abscissa indicates the fluorescence intensity of 6-FAM (6-carboxyfluorescein) in Fig. 15.

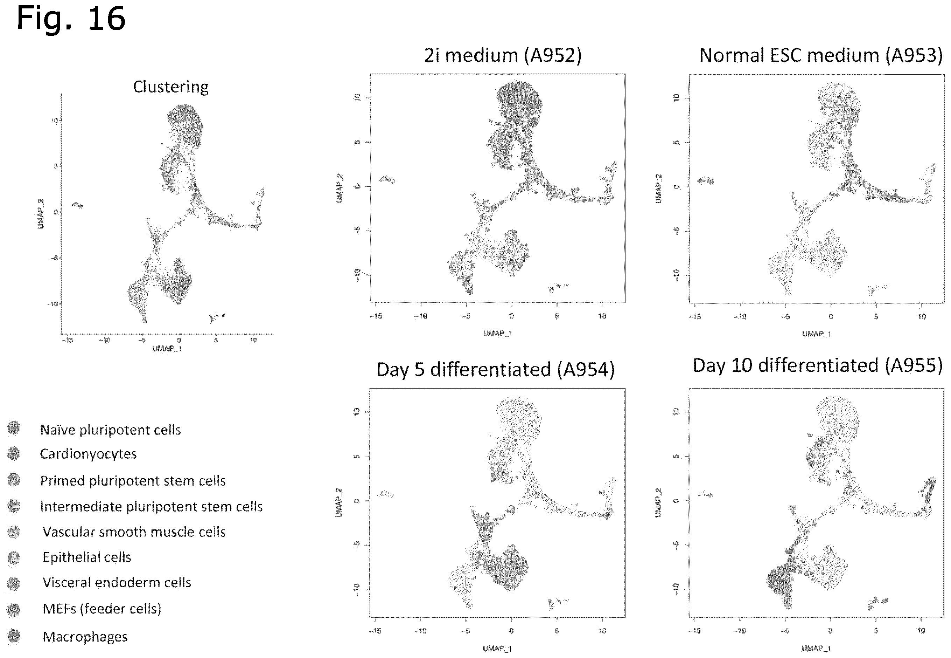

[Fig. 16] Fig. 16 shows results of multiplexed scRNA-seq analysis of various cells labeled by the single-step method.

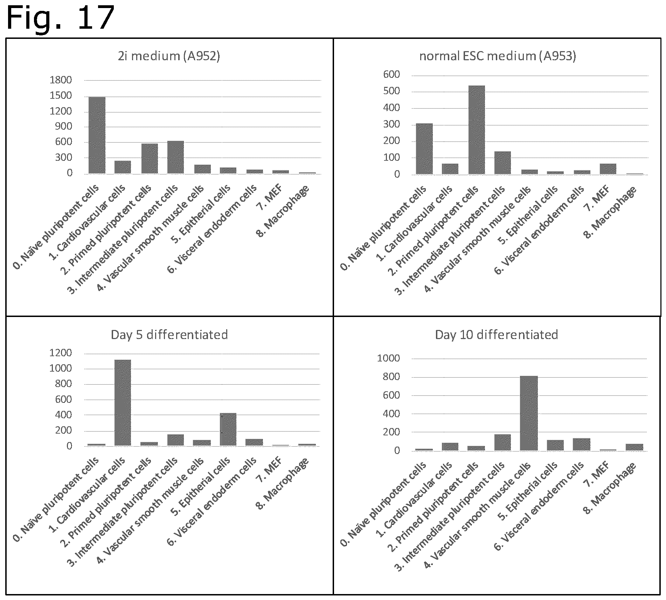

[Fig. 17] Fig. 17 is bar graphs showing the cell count of each of the cells in 9 categories detected by the multiplexed scRNA-seq analysis of Fig. 16.

### DESCRIPTION OF EMBODIMENTS

The present invention non-limitingly includes the following aspects. Unless otherwise stated herein, technical and scientific terms used herein have the same meanings as commonly understood by those skilled in the art. The substances, materials and examples disclosed herein are merely exemplary, and not intended to be limiting. When mentioning "in an aspect" herein, it means it is not limited to the aspect, in other words, is not limiting.

In an aspect, the present invention relates to a method for labeling a cell with a barcode. Non-limitingly, the method of the present invention includes:
(1) for a cell group containing a plurality of single cells, directly or indirectly bringing a cell surface protein of the cell into contact with a modified barcode; or
(2) directly or indirectly bringing a cell surface protein of a single cell into contact with a modified barcode.

The method of the present invention has characteristics, one of which is directly or indirectly bringing a cell surface protein of a cell into contact with a modified barcode.

In one aspect, the present invention is a universal surface biotinylation (USB) method. In the USB method, cell surface proteins of a cell are indirectly (via biotin-biotin binding substance) brought into contact with a barcode. In the USB method, the cell surface proteins are biotinylated, and the barcode is modified with the biotin binding substance. In the USB method, the "modified barcode" means a barcode to which a biotin binding substance is added, in other words, a barcoded biotin binding substance.

In another aspect, the method of the present invention is a method for directly binding a barcode to cell surface proteins of a cell (single-step method (or direct method)). In the single-step method, cell surface proteins of a cell are directly brought into contact with a modified barcode. In the single-step method, a barcode is modified with a compound that binds to an amino group of a cell surface protein of the cell. In the single-step method, the "modified barcode" means a barcode modified with a compound that binds to an amino group of a cell surface protein of the cell.

### 1. Method for Labeling Cell with Barcode (1) (USB method)

In an aspect, the present invention relates to a method for labeling a cell with a barcode. Non-limitingly, the method includes:
(ia) for a cell group containing a plurality of single cells, biotinylating cell surface proteins of the cell;
(ib) biotinylating cell surface proteins of a single cell; or
(ic) biotinylating cell surface proteins of a plurality of cells followed by separating the biotinylated cells into cell groups containing a plurality of single cells, or into a single cell,
   and
(ii) bringing each cell group or each single cell into contact with a barcoded biotin binding substance.

The method includes biotinylating cell surface proteins present on a cell surface. Biotinylation is applicable to any cell surface protein and capable of comprehensively labeling cells without dependent on cell type. In other words, since labeling does not depend on a specific cell surface protein, the cell type to be the subject of the method is not limited. All cells, including animal cells, plant cells, and cultured cells are to be the subject.

First, the method includes biotinylating cell surface proteins of the cell.
(ia) for a cell group containing a plurality of single cells, biotinylating cell surface proteins of the cell;
(ib) biotinylating cell surface proteins of a single cell; or
(ic) biotinylating cell surface proteins of a plurality of cells followed by separating the biotinylated cells into cell groups containing a plurality of single cells, or into single cells.

Biotinylation of proteins may be performed on a cell group containing a plurality of single cells or a single cell; alternatively, cell surface proteins of a plurality of cells may be biotinylated followed by separating the biotinylated cells into cell groups containing a plurality of single cells, or into single cells. (ii) Before the step of bringing into contact with the barcoded biotin binding substance, the cells are required to be separated into cell groups containing a plurality of single cells, or into single cells. Preferably, biotinylation of proteins is performed on the cell group containing a plurality of single cells or on a single cell.

The "cell group containing a plurality of single cells" are, for example, cell groups of mass of single cells which properties or origin are common, such as a cell group consisting of a plurality of single cells having common properties, and a cell group consisting of a plurality of single cells obtained from the common organ.

In an aspect, the method includes:
(ia) for a cell group containing a plurality of single cells, biotinylating cell surface proteins;
   and
(ii) bringing each cell group into contact with a barcoded biotin binding substance.

As the method for separating the mass of cells containing a plurality of cells (e.g., organ and the like) into cell groups containing a plurality of single cells, or into single cells, any known method can be used without particular limitation. Depending on the mass of the type, the cell type contained in the mass of cells, the cell group containing a plurality of single cells to be obtained (to be labeled) and to be separated, or the type of single cell, those skilled in the art can use a known method. The same applies to the case of separating after biotinylation.

Biotin is a water-soluble vitamin classified as vitamin B, and also referred to as vitamin B₇. Biotinylation means the process of binding biotin to proteins or macromolecules.

Examples of biotinylation include a method of chemically binding an amino group, a sulfhydryl group, a carboxyl group, or an aldehyde group (oxidation of sugar chain) to a functional group on a protein, using a biotin labeling reagent. Many types of biotin labeling reagents are known, and those skilled in the art can select an adequate biotin labeling reagent based on the functional group to be used, the hydrophilicity of the labeling reagent, presence or absence of the cleavage after labeling reaction, or the like.

Amino groups (mainly, primary amines: -NH₂) are present at a side chain or an amino terminus of lysine residues contained in many proteins. Since, in particular, the ε-amino group of a lysine residue is highly reactive, and in many cases, lysine residues are present on the surface of protein tertiary structure, the amino groups are functional groups that are particularly easy to be used for biotinylation. The representative of amino group-reactive biotin labeling reagents is biotin with an N-hydroxysuccinimide (abbreviation: NHS)-ester. NHS-esters react with amino groups to form amide bonds. NHS-ester biotin includes sulfo-NHS-ester biotin that has a sulfonic acid group, and NHS-ester biotin that does not have a sulfonic acid group. The former includes, for example, sulfo-N-hydroxysuccinimide-biotin, and the latter includes, for example, N-hydroxysuccinimide-biotin.

In addition, those with a long spacer between biotin and NHS-ester can also be used. The length of a spacer is non-limitingly 0 to 300 Å, and preferably 10 to 50 Å.
Alternatively, those with polyethylene glycol (PEG) introduced in a spacer part (e.g., NHS-PEG4-biotin) can also be used. Alternatively, those in which biotin can appropriately be cut off can also be used. In some cases, the bond between biotin and a labeled protein can be cleaved by a spacer.

Additionally, pentafluorophenyl-biotin, biotinylated isothiocyanate, and the like can be used for biotinylation of amino groups. Pentafluorophenyl-biotin and biotinylated isothiocyanate can bind to both primary amines and secondary amines. Sulfhydryl groups (-SH) of cysteine residues can also be used for biotinylation. In particular, when an amino group is present at an active site of a protein, and there is a concern that the protein is inactivated due to labeling of the amino group, or the like, the sulfhydryl group may be biotinylated. However, when performing labeling reaction, the sulfhydryl group needs to be in a reduced state, in other words, it needs not to form a disulfide bond (S-S bond) by oxidation. When any sulfhydryl group in a reduced state is not present, the disulfide bond is cleaved by a reducing agent. When the cell surface protein does not contain cysteine residues, lysine residues may be modified to generate a sulfhydryl group for use. As the biotin labeling reagent that is reactive to a sulfhydryl group, for example, biotin with a maleimide group, biotin with a bromoacetamide group, or the like can be used.

A carboxyl group (-COOH) is present at a carboxyl terminus, as well as at a side chain of aspartic acid or glutamic acid. When targeting a carboxyl group, biotin with an amino group (-NH₂) or hydrazide-derivatized biotin is reacted via a crosslinker with a carbodiimide group to form an amide bond. As the crosslinker with a carbodiimide group, for example, 1-ethyl-3-[3-dimethylaminopropyl]carbodiimide hydrochloride (EDC) or the like can be used.

Cis-diol of sialic acid on a sugar chain generates an aldehyde group (-CHO) by mild oxidative cleavage using sodium periodate (NaIO₄), for example. The aldehyde group may be reacted with hydrazide to form hydrazone bond. Mildly oxidizing the glycoprotein to generate an aldehyde group can lead hydrazide-derivatized biotin to be labeled.

Alternatively, using photoreactive biotin compounds, which non-specifically react upon exposure ultraviolet (UV) light, can also lead to non-specific biotinylation. An example thereof is use of a photoreactive biotin labeling reagent having an arylazide group. Arylazide groups are activated upon ultraviolet light irradiation, and an aryl nitrene (half-life is 10⁻⁴ seconds) non-specifically reacts with a high electron density region of double bonds or hydrogen bonds present in the vicinity, or with an amino group or a sulfhydryl group. The use of photoreactive biotin compounds is useful in a case where a non-specific labeling is required, or the like.

In an aspect, an amino group, a sulfhydryl group, or a carboxyl group of a cell surface protein is biotinylated in (i).

In an aspect, an amino group of a cell surface protein is biotinylate. In an aspect, a reagent used for biotinylation in (i) (biotin labeling reagent, reagent for biotinylating a cell surface protein of a cell) is selected from the group consisting of sulfo-N-hydroxysuccinimide-biotin, N-hydroxysuccinimide-biotin, and pentafluorophenyl-biotin.

The binding of biotin with sulfo-N-hydroxysuccinimide, N-hydroxysuccinimide, or pentafluorophenyl can include a spacer. The length of a spacer is non-limitingly 0 to 300 Å, and preferably 10 to 50 Å.

In an aspect,
a reagent used for biotinylation in (i) is selected from the group consisting of sulfo-N-hydroxysuccinimide-biotin, N-hydroxysuccinimide-biotin, and pentafluorophenyl-biotin,
wherein the binding of biotin with sulfo-N-hydroxysuccinimide, N-hydroxysuccinimide, or pentafluorophenyl can include a spacer.

In an aspect, the concentration of the reagent used for biotinylation can be appropriately applied according to the cell type used. The concentration of S-NHS-biotin in Examples was 10 µg/mL; however, it can be set to, for example, 1 to 50 µg/mL according to the cell type used.

In an aspect of the method, the reagent used for biotinylation in (i) may be those that has been cryopreserved. For example, as sulfo-N-hydroxysuccinimide-biotin, those preadjusted and cryopreserved can be used at an appropriate time.

The method above biotinylates cell surface proteins by step (i), followed by (ii) bringing each cell group or each single cell into contact with a barcoded biotin binding substance.

The "biotin binding substance" is not particularly limited as long as it has biotin binding properties. Preferred is a substance that less affects the cell growth and survival, or does not affect the cell growth and survival, due to the binding to cell surface proteins via biotin.

Examples of the biotin binding substance is non-limitingly include streptavidin, avidin, and anti-biotin antibody. In an aspect, the biotin binding substance is selected from the group consisting of streptavidin, avidin, and anti-biotin antibody. In an aspect, the biotin binding substance is streptavidin.

The "barcode" is a substance that includes information for identifying each cell group or each single cell. The type of the barcode is not particularly limited, as long as the barcode is a substance that can be added to a biotin binding substance, and includes information for identifying each cell group or each single cell. Examples of the barcode non-limitingly includes oligo DNA and oligo RNA. In an aspect, the barcode is oligo DNA.

The "barcoded biotin binding substance" is a substance in which a barcode (e.g., oligo DNA) is added to a biotin binding substance (e.g., streptavidin). To the barcoded biotin binding substance, labeling for detection can further be added. For example, by adding a fluorescent substance to the barcoded biotin binding substance, labeled cells can be analyzed by flow cytometry. Alternatively, by adding an epitope containing a specific protein or the like to the barcoded biotin binding substance and detecting with fluorescence or the like using an antibody that binds to the epitope or the like, immunofluorescence analysis can be performed.

The method of bringing each cell group or each single cell into contact with the barcoded biotin binding substance is not particularly limited. For example, it is performed by suspending each cell group or each single cell to which biotin is added, in a solution containing the barcoded biotin binding substance.

In an aspect of the method above, cells to be labeled may be viable cells ex vivo or in vivo. Alternatively, the cells to be labeled may be cells that have been fixed prior to biotinylation. In an aspect of the method above, the cells to be labeled may be cells that have been fixed and stored prior to biotinylation. The fixation and storage of cells can be performed by a known method according to the cell type. For example, cells in which methanol-fixed samples is stored at a low temperature (e.g., -80°C) can be used. Alternatively, the cells may be those that have been cryopreserved prior to biotinylation. The cryopreservation of cells can be performed by a known method according to the cell type.

The method above may be any of in vivo, ex vivo, and in vitro. Preferred is a method in vitro.

### 2. Kit (1)

In an aspect, the present invention also relates to a kit for use in a method for labeling cells with barcodes. Non-limitingly, the kit includes:
a reagent for biotinylating a surface protein of the cell, and
a barcoded biotin binding substance.

In an aspect, the kit can be used in "1. Method for Labeling Cell with Barcode (1)" or "3. Method for Multiplexed Analysis of Cell Samples (1)"

The "reagent for biotinylating a cell surface protein of a cell (reagent used for biotinylation, biotin labeling reagent)", the "barcode", the "biotin binding substance", and the "barcoded biotin binding substance" are as described in "1. Method for Labeling Cell with Barcode (1)".

In an aspect, the reagent for biotinylating the cell surface protein of the cell is sulfo-N-hydroxysuccinimide-biotin.

In an aspect, the "barcode" is oligo DNA. In an aspect, the "biotin binding substance" is streptavidin.

### 3. Method for Multiplexed Analysis of Cell Samples (1)

The present invention also relates to a method for multiplexed analysis of cell samples. Non-limitingly, the method includes:
(ia) for a cell group containing a plurality of single cells, biotinylating cell surface proteins of the cell;
(ib) biotinylating cell surface proteins of a single cell; or
(ic) biotinylating cell surface proteins of a plurality of cells followed by separating the biotinylated cells into cell groups containing a plurality of single cells, or into a single cell,
(ii) bringing each cell group or each single cell into contact with a barcoded biotin binding substance,
(iii) mixing all or a part of the cell groups or single cells barcode-labeled in (ii), and
(iv) analyzing the mixture of the cells of (iii).
(ia), (ib), (ic), and (ii) are as described in "1. Method for Labeling Cell with Barcode (1)".

The method for multiplexed analysis of cell samples described above includes, after step (ii),
(iii) mixing all or a part of the cell groups or single cells barcode-labeled in (ii),
and
(iv) analyzing the mixture of the cells of (iii).

In the method, since labeling is performed with a barcode that can identify each cell group or each single cell, after labeling, a mixture of all or a part of the cell groups or single cells barcode-labeled can be collectively analyzed. In other words, even if analysis is collectively performed, it is possible to identify which results are from which cell group or single cell, by a barcode that can identify each cell group or each single cell.

The analysis is not limited as long as it is means for analyzing cells. Examples to be analyzed include the cell content (e.g., nucleic acids such as DNA and RNA). In an aspect, the analysis is cellular RNA analysis.

Flow cytometry, immunofluorescence analysis or the like can be performed by directly or indirectly (secondarily) labeling the barcoded biotin binding substance with fluorescence or the like.

Alternatively, by using a uniform manifold approximation and projection (UMAP) method or the like based on data obtained by scRNA-seq analysis, cells with similar nature can be classified into a plurality of clusters. UMAP is an analysis tool that performs dimension reduction (dimension compression) on a given point sequence and visualizes points that are "close" to each other in a high dimension by disposing them "close" to each other in a low dimension as well. Differences in properties of each sample can be analyzed, for example, by comparing the difference between the cell counts of the samples contained in each cluster, classified by identifying samples that are origins using barcodes different for each sample as an index.

Also, the mixture of all or a part of the cell groups or single cells barcode-labeled can be individually analyzed by collectively sequencing and distinguishing which sequence is for which cell group or each single cell by barcode identification.

In addition to RNA analysis, application to genomic DNA analysis is also conceivable. For example, it becomes possible to create cell division lineage for each cell by genomic DNA sequencing in single cells to detect DNA base sequence mutation for each single cell. Sample multiplexing by adding barcodes when genomic DNA sequencing in single cells makes it possible to significantly reduce analysis costs for creating cell division lineage from single cell groups derived from a plurality of tissues. In addition, for example, the quantitative detection of mitochondrial DNA by DNA sequencing in single cells makes it possible to trace variation in the number of mitochondria in cell groups. In this case, sample multiplexing by adding barcodes makes it possible to reduce analysis costs.

In an aspect, the method for multiplexed analysis of the cell samples includes:
(ia) for a cell group containing a plurality of single cells, biotinylating cell surface proteins;
   and
(ii) bringing each cell group into contact with a barcoded biotin binding substance.

The present inventors have newly developed a simple yet reliable method for labeling cells for multiplexing in scRNA-seq analysis. Conventional cell hashing methods target specific proteins that are widely expressed on the surface of the cell tested, and are therefore difficult to be used when such proteins are not widely expressed in samples. In contrast, the USB method does not require such specific proteins, and overcomes disadvantages of existing technologies by using a universal label that can target any protein.

Multiplex reagents that are currently available, use antibodies against cell surface proteins such as CD298 and/or MHC Class I antigens or CD45. These antigens are thought to be universally expressed in adult tissues or the immune system. However, embryonic cells do not express these proteins, and thus the current cell hashing method is not applicable. Contrary to this, the USB method has applicability to all cell surface proteins, and can be used for multiplexing regardless of cell type (as long as cell surface proteins exist). In fact, to the multiplexing of rat lung cells, which was not able to be studied by the Ab method that uses chondrocytes derived from iPS cells of mouse, monkey or human, and CD45 or MHC antibodies, the USB method was successfully applied (Figs. 11 and 12).

Conventional labeling techniques for multiplexing, such as the transient barcoding method that introduces barcoded DNA into cells, and CellTag indexing method that uses a lentivirus for barcoded DNA introduction, are difficult to be applied to cells other than cultured cells, and therefore, its versatility is limited. In contrast, the USB method is highly versatile and applicable to any type of cell.

The USB method is also applicable to animals other than mouse or human. For other species. the information on cell surface proteins or antibodies against them may not be readily available, and thus the conventional Ab method is difficult to be applied thereto. However, the USB method has been proved to be applicable to monkeys and rats, and is theoretically applicable to cells of any species.

Further, the ClickTag multiplexing method in which click chemistry and NHS-ester are combined (NPL 13) is a technique for attaching a barcoded DNA tag to a cell protein, as is the USB method. However, the labeling efficiency of viable cells is poor, and this method can only be applied to methanol fixed cells. The CellPlex method (3' CellPlex Kit, 10x Genomics) uses a reagent that introduces a DNA tag into lipid in the cell membrane for universal cell labeling (NPL 14), but it is difficult to be applied to methanol fixed cells. Contrary to this, the USB method is also applicable to fixed cells, and enabling more flexible sample multiplexing.

As described in the section "Materials and Methods" in EXAMPLES, all components used in the USB method are commercially available and relatively inexpensive. The stock solution of S-NHS-biotin is stable at -80°C for more than 1 year, and a vial containing 50 mg of S-NHS-biotin is sufficient for labeling up to 10,000 samples.

10 micrograms of DNA-barcoded streptavidin can be used for no less than 150 multiplexing experiments. Since 10 types of DNA-barcoded streptavidin are commercially available, 10-plex analysis can be performed at once. Streptavidin to which arbitrary barcoded oligo DNA is added can be custom-made, and thus 10-plex multiplexing experiments or more can also be performed. That is, the USB method is a highly cost-effective method, which makes SINGLE CELL multiplexing analysis more affordable, and the USB method is a universal labeling method that is applicable to both viable cells and fixed cells, and that makes all kinds of multiplexed scRNA-seq analysis greatly easy.

Comparing to the single-step method, the USB method is characterized by the fact that it is easy to be newly introduced because existing reagents can be used, the cost per sample is lower than the single-step method, and the like.

The method above may be any of in vivo, ex vivo, and in vitro. Preferred is a method in vitro.

### 4. Method for Directly Labeling Cell with Barcode (2) (Single-Step Method).

In an aspect, the present invention relates to a method for labeling a cell with a barcode. Non-limitingly, the method includes:
(1A) bringing a cell group containing a plurality of single cells into contact with a barcode modified with a compound that binds to an amino group of a cell surface protein of the cell; or
(1B) bringing a single cell into contact with a barcode modified with a compound that binds to an amino group of a cell surface protein of the cell,

The "cell group containing a plurality of single cells", "cell", "barcode", "cell surface protein", and the like are as described in "1. Method for Labeling Cell with Barcode (1)". By bringing a cell into contact with a barcode modified with a compound that binds to an amino group of a cell surface protein of the cell, the compound that binds to an amino group of surface proteins in the modified barcode binds to an amino group of cell surface proteins, and then the cell is labeled with the barcode.

In an aspect, the compound that binds to the amino group of the cell surface protein of the cell is selected from the group consisting of sulfo-N-hydroxysuccinimide, N-hydroxysuccinimide, and pentafluorophenyl.

Amino groups (mainly, primary amines: -NH₂) are present at a side chain or an amino terminus of lysine residues contained in many proteins. Since, in particular, the ε-amino group of a lysine residue is highly reactive, and in many cases, lysine residues are present on the surface of protein tertiary structure, the amino groups are functional groups that are particularly easy to be used for protein modification. The representative of the compound that binds to the amino group of the cell surface protein of the cell is N-hydroxysuccinimide (abbreviation: NHS)-ester. An NHS-ester barcode reacts with the amino group of the cell surface protein to form an amide bond. NHS-esters includes sulfo-NHS-ester that has a sulfonic acid group, and NHS-ester that does not have a sulfonic acid group. The former includes, for example, sulfo-N-hydroxysuccinimide (sulfo-NHS), and the latter includes, for example, N-hydroxysuccinimide (NHS).

In addition, a spacer can be provided between the barcode and NHS-ester. The length of a spacer is non-limitingly 0 to 300 Å, and preferably 10 to 50 Å. Alternatively, those with polyethylene glycol (PEG) introduced in a spacer part (e.g., NHS-PEG4) can also be used.

The method for binding a barcode and NHS-ester is not particularly limited, and can be performed by any method.

In an aspect, a carbodiimide compound, such as 1-ethyl-3-[3-dimethylaminopropyl]carbodiimide hydrochloride (EDC), N,N'-dicyclohexylcarbodiimide or the like is used. Incorporation of sulfo-NHS or NHS into EDC binding protocol makes reaction efficiency to be raised, and a dry-stable (amine-reactive) intermediate is generated. Specifically, first, oligo DNA (barcode) modified with a carboxyl group (-COOH) at the 5' end is prepared, for example. The addition of a carboxyl group to oligo DNA can be performed by a known method. For example, it can be non-limitingly performed by using 10-carboxy-decyl-(2-cyanoethyl)(N,N-diisopropyl)-phosphoramidite, or N-hydroxysuccinimide ester (e.g., reagent, 5'-Carboxy-Modifier C10 (Glen Research). Next, NHS is coupled to -COOH of 5'-COOH-DNA by EDC to form an extremely highly stable NHS-ester relative to O-acylisourea intermediate. DNA that has been "(sulfo-) NHS-esterified" (activated state) form a stable bond with the amino group in the cell surface proteins. This allows cells to be labeled by the (sulfo-) NHS-esterified DNA (modified barcode).

Alternatively, the "(sulfo-) NHS-esterified" DNA may be made by the following method.

For example, the "(sulfo-) NHS-esterified" DNA is induced by reacting -NH₂-modified oligo DNA with disuccinimidyl suberate (DSS) (Thermo Fisher Scientific (TM), 21655 and the like) or bis(succinimidyl) suberate (BS3) (Thermo Fisher Scientific (TM), 21580, and the like). Specifically, first, oligo DNA (barcode) modified with an amino group (-NH₂) at the 5' end is prepared, for example. The addition of -NH₂ to DNA can be performed by a known method. For example, it can be non-limitingly performed by using 6-(4-monomethoxytritylamino)hexyl-(2-cyanoethy)-(N,N-diisopropyl)-phosphoramidite (e.g., reagent, 5'-Amino-Modifier C6 (Glen Research)). Next, DNA with -NH₂ added at the 5' end is reacted with DSS or BS3. DSS is a reagent that have two NHS-esters; one NHS-ester is coupled to -NH₂ by reacting it with DNA to creates a state in which the other is exposed, and the activated NHS-ester is added to DNA terminus. BS3 is DSS with a sulfo group added, and as is DSS, one NHS-ester is coupled to -NH₂ by reacting it with DNA to creates a state in which the other is exposed, and the activated NHS-ester is added to DNA terminus. In this method, once two DNAs and one DSS or BS3 bind, they can no longer bind to cells afterwards. Therefore, adjustment of reaction conditions (reaction time, and the like) is needed so that only one DNA binds to DSS or BS3.

In addition, pentafluorophenyl, isothiocyanate, and the like also modify a barcode, and can be used for binding to the amino groups of the cell surface proteins. Pentafluorophenyl and isothiocyanate can bind to both primary amines and secondary amines.

Note that 6-FAM(6-carboxyfluorescein), biotin, and the like may be bound to a portion of the barcode that has not been NHS-ester modified, to use for detection or the like other than multiplexed analysis or the like. For example, in a case where oligo DNA is NHS-ester modified at the 5' end, 6-FAM or biotin may be added to the 3' end of the DNA.

In an aspect, the compound that binds to an amino group of cell surface proteins of the cell has been cryopreserved.

In an aspect, the barcode is oligo DNA.

In an aspect, the method is for labeling a viable cell. In an aspect, the method is for labeling a fixed cell. The "viable cell", "fixed cell", and the like are as described in "1. Method for Labeling Cell with Barcode (1)".

The method above may be any of in vivo, ex vivo, and in vitro. Preferred is a method in vitro.

The method above may be any of in vivo, ex vivo, and in vitro. Preferred is a method in vitro.

In addition, unless there are any technical problems, the content described in "1. Method for Labeling Cell with Barcode (1) (USB Method)" also applies to "4. Method for Directly Labeling Cell with Barcode (2) (Single-Step Method)" in this section.

The single-step method directly brings cell surface proteins of a cell into contact with a modified barcode, which allows the working process to be simplified, and generally the working time is shorter than that of the USB method (2 hours of process time). A shorter working time can reduce influence on cells. Since the number of centrifugation operations is reduced, loss of the cells due to the operations can be reduced.

### 5. Kit (2)

In an aspect, the present invention also relates to a kit for use in a method for labeling cells with barcodes. The kit non-limitingly includes a barcode modified with a compound that binds to an amino group of a cell surface protein of the cell.

In an aspect, the kit can be used in "4. Method for Directly Labeling Cell with Barcode (2)" or "6. Method for Multiplexed Analysis of Cell Samples (2)".

The "method for labeling cells with barcodes", "barcode modified with a compound that binds to an amino group of a cell surface protein of the cell", and the like are as described in "4. Method for Directly Labeling Cell with Barcode (2)".

In an aspect, the compound that binds to an amino group of a cell surface protein of the cell is sulfo-N-hydroxysuccinimide.

In addition, unless there are any technical problems, the content described in "2. Kit (1)" applies to Kit (2) in this section.

### 6. Method for Multiplexed Analysis of Cell Samples (2)

The present invention also relates to a method for multiplexed analysis of cell samples. Non-limitingly, the method includes:
(1A) bringing a cell group containing a plurality of single cells into contact with a barcode modified with a compound that binds to an amino group of a cell surface protein of the cell; or
(1B) bringing a single cell into contact with a barcode modified with a compound that binds to an amino group of a cell surface protein of the cell,
(2) mixing all or a part of the cell groups or single cells barcode-labeled in (1), and
(3) analyzing the mixture of the cells of (2).
(1A) and (1B) are as described in "4. Method for Directly Labeling Cell with Barcode (2)".
(2) and (3) are as described in "3. Method for Multiplexed Analysis of Cell Samples (1)".

In an aspect, the analysis is cellular RNA analysis.

The method above may be any of in vivo, ex vivo, and in vitro. Preferred is a method in vitro.

In addition, unless there are any technical problems, the content described in "3. Method for Multiplexed Analysis of Cell Samples (1)" also applies to Method for Multiplexed Analysis of Cell Samples (2) in this section.

### EXAMPLES

The present invention will be described in detail by reference to Examples; however, the present invention is not limited to the following Examples. Those skilled in the art can easily modify or change the present invention based on the description herein, and such modification and changes are included in the technical scope of the present invention.

### Materials and Methods

Unless otherwise clearly stated, the following materials and methods were used in

### EXAMPLES.

### 1. Cell Culture

R1 ES cells were provided from Dr. Sado (Kindai University). EB3 ES cells (AES0139) (NPLs 15 and 16) were provided from Cell Engineering Division (Cell Bank) of Riken BioResource Research Center.

Undifferentiated ES cells were maintained according to the method described in NPL 17 with some modifications. Briefly speaking, ES cells were cultured in Glasgow's Minimum Essential Medium (GMEM; Merck KGaA, G5154) to which 14% Knockout Serum Replacement (KSR; Thermo Fisher Scientific (TM), 10828028), 1% fetal bovine serum (FBS), 1,000 U/mL leukemia inhibitory factor (LIF), 0.11 mg/mL sodium pyruvate (Natalai Tesque, 29806-12), 0.1 mM 2-mercaptoethanol (Thermo Fisher Scientific (TM), 21985023), 1× non-essential amino acid (Thermo Fisher Scientific (TM), 11140076) and 1× GlutaMAX supplement (Thermo Fisher Scientific (TM), 350061) were added, on mouse embryonic fibroblast feeder cells treated with mitomycin C. For passage, 0.25% trypsin (FUJIFILM Corporation, 201-16945) was used.

### 2. Differentiation Induction of ES Cells

After removing the feeder cells, 1 × 10⁴ ES cells were seeded in 100 µL differentiation medium (GMEM supplemented with 15% FBS, 0.11 mg/mL sodium pyruvate, 0.1 mM 2-mercaptoethanol, 1× non-essential amino acid, and 1× GlutaMAX supplement) in a U-bottom 96-well low cell attachment plate (Nunclon Sphera 96-Well U-Shaped-Bottom Microplate; Thermo Fisher Scientific (TM), 174925), and cultured for 24 hours. Spheroids made in the plate were transferred to a 90 mm, low cell attachment culture dish (Nunclon Sphera 90 mm dish, 174945), and cultured for further 4 days to form embryoid bodies (EBs). The EBs were washed three times with PBS, dissociated into SINGLE CELLs (group of single cells) using 0.25% trypsin, the cells were seeded on a gelatin-treated culture dish, and cultured for 7 days to induce further differentiation.

### 3. Preparation of SINGLE CELLs for scRNA-seq Analysis

ES cells were dispersed into SINGLE CELLs, cultured for 1 hour on the gelatin-treated culture dish, and the feeder cells were removed. Suspension cells were collected as undifferentiated ES cells.

Differentiated ES cells were washed three times with PBS, treated with 0.25% trypsin, and then dispersed into SINGLE CELLs. Dead cells contained in a cell suspension solution were removed by Percoll centrifugation method. Briefly speaking, the cells were suspended in a 25% Percoll PLUS solution (Cytiva, Inc., 17544501) which concentration was adjusted by using RPMI 1640 (FUJIFILM Corporation, 183-02165) added with 5% FBS and 10 mM HEPES (Natalai Tesque, 17557-94), and layered on a 65% Percoll PLUS solution. After centrifugation at 1,000 g for 20 minutes, the interphase was gathered and washed with differentiation medium. The cell suspension solution was filtered through 40 µm cell strainer, and trypan blue-unstained cell percentage was measured as cell viability.

### 4. USB Labeling

A S-NHS-biotin stock solution was prepared by dissolving EZ-Link sulfo-NHS-biotin (Thermo Fisher Scientific (TM), 21217) in PBS at a concentration of 10 mg/mL, and dispensed and stored at -80°C until use.

Single cells containing 0.5-2 × 10⁶ SINGLE CELLs prepared in "3. Preparation of SINGLE CELLs for scRNA-seq Analysis" were washed once with ice-cold PBS added with 1% FBS. The washed cells were suspended in 500 µL ice-cold NHS-biotin working solution in PBS added with 1% FBS, and incubated on ice for 10 minutes. The concentration of S-NHS-biotin in EXAMPLES was 10 µg/mL.

After incubation on ice, 3 mL ice-cold PBS containing 3% FBS was added to the reaction, and the cells were centrifuged at 4°C, 300 g for 5 minutes. The cells were further washed twice with Cell Staining Buffer (BioLegend, Inc., 420201), and then kept on ice until barcoded oligo DNA binding in the next step.

### 5. Antibody Labeling with Anti-CDH1 Antibody (Control Experiment)

Single cells containing 0.5-2 × 10⁶ SINGLE CELLs prepared in "3. Preparation of SINGLE CELLs for scRNA-seq Analysis" were washed once with ice-cold Cell Staining Buffer, suspended in 50 µL ice-cold blocking solution (0.05 µg/mL TruStain FcX PLUS antibody [BioLegend, Inc., 156603] in Cell Staining Buffer), and incubated on ice for 10 minutes. An ice-cold primary antibody solution (0.05 µg/mL biotinylated CDC324 [E-cadherin] rat monoclonal antibody [Thermo Fisher Scientific (TM), 13-3249-82]) was mixed with the cell suspension solution, and the cell was incubated on ice for 30 minutes. Next, the cells were washed three times with ice-cold Cell Staining Buffer, and then kept on ice until binding of barcoded DNA.

As an isotype control, biotinylated rat IgG1 isotype control (Thermo Fisher Scientific (TM), 13-4301-82) was used.

### 6. Barcoded Oligo DNA Binding.

By using 5 types of TotalSeq (R) PE streptavidin (BioLegend, Inc., 405251, 405253, 405255, 405257, 405259) or TotalSeq (R) anti-biotin antibody (BioLegend, Inc., 409008), the biotin-labeled cells prepared in "4. USB Labeling" or the antibody-labeled cells prepared in "5. Antibody Labeling with Anti-CDH1 Antibody (Control Experiment)" were allowed to bind to barcoded oligo DNA as follows. The information on the barcoded oligo DNA used is shown in the table below.

**[Table 1]**

| Tag ID in this research | Reagent name | Cat. No. | Barcode oligo DNA sequence |
|---|---|---|---|
| A951 | TotalSeq^{™}-A0951 PE Streptavidin | 405251 | AACCTTTGCCACTGC |
| A952 | TotalSeq^{™}-A0952 PE Streptavidin | 405253 | GTCCGACTAATAGCT |
| A953 | TotalSeq^{™}-A0953 PE Streptavidin | 405255 | CAGGTTGTTGTCATT |
| A954 | TotalSeq^{™}-A0954 PE Streptavidin | 405257 | TATTTCCACCCGGTC |
| A955 | TotalSeq^{™}-A0955 PE Streptavidin | 405259 | GTTGTGAGCACGAGA |
| A436 | TotalSeq^{™}-A0436 anti-Biotin Antibody | 409008 | CGGTATATCAACAGA |

| | | | |
|---|---|---|---|
| (SEQ ID Nos: 1-6) | | | |

The cells were suspended in 100 µL solution containing 0.6 µg/mL ice-cold TotalSeq (R) PE streptavidin or 2 µg/mL TotalSeq (R) anti-biotin antibody, diluted with Cell Staining Buffer, incubated on ice for 30 minutes, and then washed three times. The cell suspension solution was filtered through 40 µm cell strainer, the same number of cells from each sample was gathered in a single tube and used for cell trapping and cDNA synthesis using a BD Rhapsody (TM) apparatus.

### 7. Flow Cytometry

First, by the same method as in the above 1-3, SINGLE CELL suspension solutions of undifferentiated ES cells and differentiated ES cells were prepared.

Specifically, healthy F344 rat lungs were cut into 0.5 mm² with razor blade, digested in a Liberase solution [RPMI-1640 (Natalai Tesque)] added with 10% FBS, 10 mM HEPES pH 7.2-7.4, 0.25 mg/ml Liberase (TM) (F. Hoffmann-La Roche Ltd.), and 2000 U/mL DNase I (Merck KGaA) for 60 minutes at 37°C to prepare a SINGLE CELL suspension solution. Dead cells were removed by Percoll. Alternatively, growth plate cartilage from proximal tibia was collected from 3-week-old mice and cut finely into 1 to 2 mm pieces. Next, the cut pieces were dispersed into SINGLE CELLs (group of single cells) with Liberse TM (F. Hoffmann-La Roche Ltd.) solution for 120 to 210 minutes while continuously agitating.

Reagents for cell staining were biotinylated CDC324 (E-cadherin) rat monoclonal antibody (Thermo Fisher Scientific (TM), 13-3249-82), TotalSeq (R) PE streptavidin (BioLegend, Inc., 405251), PerCP-Cy5.5 anti-rat CD45 (Bio-Rad Laboratories, Inc., clone OX-1), PE anti-rat CD31 (Bio-Rad Laboratories, Inc., Clone TLD-3A12), streptavidin-APC (BioLegend, Inc., 405207), APC rat anti-mouse CD45 (BD Biosciences, 559864), and PE anti-mouse H-2 antibody (BioLegend, Inc., 125505).

The stained cells were analyzed by flow cytometry using EC800 Cell Analyzer (Sony Corporation), Cyto FLEX flow cytometer (Beckman Coulter, Inc.), or FACS Arria II flow cytometer (BD Biosciences).

All animal experimentation was reviewed and approved by the Animal Committee in Kyoto University, and the Animal Care and Use Committee of Tokyo University of Science (approval numbers: S17034, S18029, S19024, and S20019).

### 8. Immunofluorescence Method

Cells cultured in gelatin-treated culture dish were fixed with 4% paraformaldehyde in PBS overnight at 4°C, and then washed three times with washing buffer solution (PBS containing 0.1% Triton X-100). The cells were incubated in a blocking buffer solution (PBS containing 1% BSA and 0.1% Triton X-100), and then incubated overnight with a primary antibody diluted in the blocking buffer solution.

After washing with the washing buffer solution, the cells were incubated for 1 hour at room temperature with a secondary antibody diluted in the blocking buffer solution. Next, the cells were mounted with a solution adjusted to 2% DABCO (Sigma Aldrich Co. LLC, D2522) with PBS containing 40% glycerol. Images were captured with LEICA AF6500 fluorescent imaging system (Leica Microsystems).

The antibodies and dilution ratios used in EXAMPLES were as follows.

Primary antibody- biotinylated CDC324 (E-cadherin: CDH1) rat monoclonal antibody (Thermo Fisher Scientific (TM), 13-3249-82) (1:200) and anti-OCT4 rabbit polyclonal antibody (Abcam plc, ab19857) (1:400);

Secondary antibody- Alexa Fluor 488 anti-rat donkey IgG (Thermo Fisher Scientific (TM), A21208) (1:500) and Alexa Fluor 555 anti-rabbit donkey IgG (Thermo Fisher Scientific (TM), A31572) (1:500).

### 9. Cell Viability Test

Undifferentiated ES cells were treated with 100 µg/mL S-NHS-biotin (10-fold the concentration used for multiplex scRNA-seq) and TotalSeq (R) PE streptavidin, and 3 × 10³ cells were seeded on a 3.5 cm, gelatin-treated culture dish. Concurrently, the same number of untreated cells were seeded. After 5-day culture, the cells were stained with hematoxylin, and the colony formation ratios of untreated control and treated sample were examined.

### 10. scRNA-seq

For preparation of a scRNA-seq library, a part of BD Rhapsody Express Single-cell Analysis system (BD Biosciences) and Targeted mRNA and AbSeq Amplification Kit (BD Biosciences, 633771) was used.

Cells (1.6 × 10⁴) labeled with barcoded oligo DNA were trapped in a microwell cartridge, and cell entrapping beads to which mRNA and barcoded oligo DNA derived from SINGLE CELLs were bound were collected to be used for cDNA synthesis.

The cDNA synthesis was performed according to the BD Rhapsody Express manual. cDNA and barcoded oligo DNA were amplified according to TAS-seq protocol (NPL 10). Briefly speaking, by treating with 0.75 U/µL Terminal deoxynucleotidyl transferase (TdT, Enzymatics, Inc., P7070L) solution (containing 1 mM dCTP [GE healthcare technologies Inc., 28406512] and 0.05 mM ddCTP [GE healthcare technologies Inc., 27206101] in 1 × TdT buffer solution [Thermo Fisher Scientific (TM), 16314015]) for 30 minutes while mixing at 1,200 rpm, a poly-C tail was added to the cell entrapping beads to which cDNA and barcoded oligo DNA were bound. For synthesizing a second-strand cDNA sequence, poly-C tail cDNA beads were treated by 1× KAPA HiFi HS ReadyMix (F. Hoffmann-La Roche Ltd., 7958935001) using 5'-BDWTAv2-9G primer at a program of [98°C for 20 seconds, 47°C for 1 minute, and 72°C for 2 minutes] × 16 cycles.

Next, 1× KAPA HiFi HS ReadyMix using 3 primers (5'-BDWTAv2, Universal Oligo-long, and TotalSeq-ADT-oligo 1) was added to the reaction, and the whole transcriptome was amplified using a program of [98°C for 20 seconds, 63°C for 20 seconds, and 72°C for 5 minutes] × 7 cycles. The amplified cDNA and barcoded oligo DNA were fractionated by size sorting using AMPure XP beads (Beckman Coulter, Inc., A63881).

The cDNA was further treated by 1× KAPA HiFi HS ReadyMix using 2 primers (5'-BDWTAv2 and Universal Oligo-long) using a program of [98°C for 20 seconds, 65°C for 20 seconds, and 72°C for 5 minutes] × 5 cycles. The barcoded oligo DNA was further amplified using 2 primers (TotalSeq-ADT-oligo 2 and Universal Oligo-long) using a program of [98°C for 20 seconds, 65°C for 20 seconds, and 72°C for 5 minutes] × 12 cycles.

The sequences of the primers are as shown in Table below.

**[Table 2]**

| Name | Sequence | Grade |
|---|---|---|
| 5'BDWTAv2-9G | AAGCAGTGGTATCAACGCAGAGGGGGGGGG | OPC |
| 5'BDWTAv2 | NH2-(C12)-AAGCAGTGGTATCAACGCAGAG | OPC |
| Universal Oligo-long | NH2-(C12)-ACACTCTTTCCCTACACGACGCTCTTCCGATCT | OPC |
| totalseq-ADT-oligo1 | TGCTCTTCCGATCTTGGCACCCGAGAATTCCA | OPC |
| totalseq-ADT-oligo2 | GTGACTGGAGTTCAGACGTGTGCTCTTCCGATCTTGG | OPC |

| | | |
|---|---|---|
| (SEQ ID Nos: 7-11) | | |

NH₂-C₁₂ is bound to the 5' ends of SEQ ID NOs: 8 and 9.

The sequences of the amplified cDNA and barcoded oligo DNA were purified using AMPure XP beads, and the size distribution and yields were checked by a bioanalyzer (Agilent Technologies, Inc.) using High Sensitivity DNA Kit (Agilent Technologies, Inc., 5067-4626). Sequence data were collected using NovaSeq 6000 S4 flow cell (Illumina, Inc.) at Immuno GeneTeqs, Inc.

### 11. Data Analysis

Sequence data that have been demultiplexed and DBEC-treated were imported to R (4.0.2), and the cells determined to be "doublets" or "not detected" from tag count analysis and the cells with a small number of sequence reads were deleted. Also, genes with a small number of reads, and genes that were not found in a sufficient number of cells were deleted. Seurat package (4.0.5) was used for subsequent analysis, and parameters were modified according to the tutorial workflow (NPL 18). The tutorial workflow is typically "creating Seurat object by using expression table. After performing quality check of the data, normalizing to extract characteristic genes. Performing dimensional compression to form clusters of cells which are the same cell type and conditions. By cluster analysis, classifying cells in the dataset and labeling. In order to determine the cell type and annotate, confirming the marker gene and expression variable genes per cluster".

In the present EXAMPLE, cells in which mitochondrial genes accounts for more than 10% were excluded. Next, using the count matrix, LogNormalize, which is a default, global scaling normalization method, was performed. As the extraction method, vst was used. The "vst" basically detects gene in which the variance of the expression level is greater than that of the average expression level obtained in all cells. First, a straight line is fitted to the relationship of log (variance) and log (mean) using local polynomial regression (loess). As a result, the feature amount can be standardized without removing unexpected variations. Next, using the observed mean and expected variance (given by the fitted line), the feature amount is standardized. After clipping to the maximum value, the variance of the feature amount to a standardized value is calculated. In order to reduce the impact of technical outliers, the standardized value is clipped to a maximum value. The top 2,000 highly variable genes were calculated by FindVariableFeatures (command to identify a feature amount (feature, e.g., gene) which is an outlier on a "mean variability plot").

After scaling the data, PCA was performed. Based on the Euclidean distances in a space occupied by the first 30 identified principal components, K-nearest neighbor (KNN) graph was constructed, and Louvain algorithm was applied for cluster identification. The first clustering yielded 11 clusters. In addition, UMAP was performed using 2,000 highly variable genes and 30 principal components to obtain 11 subclusters. Marker genes for each cluster were identified using FindAllMarker (command to identify a gene enriched in each cluster formed, in this EXAMPLE, Wilcoxon Rank Sum test was applied as default) (min.pct = 0.25, logfc.threshold = 0.25, set.only.pos = TRUE). The expression of E-cadherin gene (Cdh1) was plotted by FeaturePlot (command to visualize "feature" on a dimension reduced plot).

### 12. Data Availability

All raw FASTQ sequence files was registered to DNA Databank of Japan (DDBJ) under the Accession numbers of DRR333192 to DRR333193.

### EXAMPLES

### EXAMPLE 1 Cell Labeling by USB Method 1.1 Principle of Cell Labeling

The present inventors have developed a novel cell labeling technique for multiplexing scRNA-seq using S-NHS-biotin named universal surface biotinylation (USB) method (Fig. 1). Compared to that the conventional method (Ab method) targets specific proteins on the cell surface, the USB method targets amine groups present in almost all proteins, and it is thus a universal method without requiring specific surface antigens. When target surface proteins are not expressed, the conventional method cannot label cells, whereas the USB method can label them as long as surface proteins are present (Fig. 1).

In this EXAMPLE, as a proof-of-concept experiment, mouse ES cells were used to compare two methods for analysis using undifferentiated cells and differentiated cells to be analyzed. Almost all mouse ES cells express adhesion molecules, E-cadherin (CDH1) (NPL 9) in the undifferentiated state; however, the number of cells that do not express CDH1 increases as the differentiation proceeds (Fig. 6). Accordingly, in an antibody assay using an anti-CDH1 antibody, most cells in the undifferentiated state are labeled, but not all cells are labeled when it comes to differentiated cells. In contrast, since the USB method does not depend on CDH1 expression, it is expected to be able to label cells both in undifferentiated and differentiated states.

### 1.2 Cell Labeling by USB

SINGLE CELLs were prepared from undifferentiated mouse ES cells (R1 line) and cells obtained by differentiation of R1 cells by embryoid body formation, and used in the following experiments.

The cell samples were divided in two; one half was treated by the Ab method, in other words, using a biotin-conjugated anti-CDH1 antibody, and the other half was added with biotin to cell surface proteins using the USB method. Since barcoded DNA-labeled streptavidin used was coupled to fluorochrome phycoerythrin (PE), flow cytometry analysis was performed to examine labeling efficiency (Fig. 2).

When using antibody labeling, the percentage of PE-positive cells in the undifferentiated ES cells was 87.8%, whereas the percentage of PE-positive cells in the differentiated ES cells was about 10%. When immunostaining was performed by using anti-CDH1 antibodies, almost all cells in the undifferentiated ES cell samples were CDH1-positive (Fig 3), but CDH1 expression was detected only in a part of cells in the differentiated ES cells, which was consistent with the flow cytometry results.

In contrast, when using the USB method, the percentages of PE-positive cells in the undifferentiated ES cells and differentiated cell samples were 99.6% and 96.4%, respectively (Fig. 2 and 3). Also, by the fluorescence microscope observation, almost all cells were shown to be fluorescently labeled (Fig. 3, upper). These results show that the USB method is able to efficiently label most cells both in the undifferentiated and differentiated states.

### EXAMPLE 2 Operation of USB Method and Cell Viability

This EXAMPLE revealed that the operation of the USB method does not affect cell viability.

A series of experimental operations by the USB method has a possibility to compromise cell states and growth. To examine this possibility, ES cells were treated by the USB method, and seeded on a culture dish to measure colony formation ability (Fig. 7). As a result, even though cells were treated with S-NHS-biotin at a concentration 10-fold the concentration used in the USB method (0.1 mg/mL), the difference of colony formation rates between the control group without labeling and the USB group were not observed. Consequently, the USB method was concluded to not change the cell viability.

### EXAMPLE 3 Examples of Sample Multiplexing and scRNA-seq Analysis by USB method

The USB method of the present invention can comprehensively bind DNA barcodes to most various cells through biotin-streptavidin binding, and its experimental operations does not cause the cell viability to be compromised. In this EXAMPLE, examined were whether the USB technique can be used for multiplexing in scRNA-seq analysis, and whether the expression profile of the cells are affected by the experimental operations.

R1 ES cells in the undifferentiated and differentiated states were treated by either the USB method or the Ab method. Further, undifferentiated and differentiated cells of another ES cell line, EB3, were separately labeled using the USB method. Equal amounts of these 6 samples were mixed, and approximately 16,000 SINGLE CELLs were trapped using the BD Rhapsody (TM) system. Some of these cells (about 4,000 cells) were used for cDNA synthesis and amplification for the whole transcriptome analysis, as well as amplification of barcoded oligo DNA tags according to the TAS-seq protocol (NPL 10). When estimating the size distribution and yields of the amplified cDNA and barcoded oligo DNA, appropriate amplification was confirmed (Fig. 8).

After removing dead cells, doublet cells and cells for which no tag could be identified, the remaining 2,089 cells were subjected to informatics analysis (Table 3).

**[Table 3]**

| Tag ID | Cells | Labeling methods | Total cell no. | E-Cad(+) cell no. (Clusters 1, 2, 3 and 5) | E-Cad(-) cell no. (Clusters 0, 4, 6, 7, 8, 9 and 10) |
|---|---|---|---|---|---|
| A951 | R1 undiff. ESC | USB | 339 | 310 (85.9%) | 51 (14.1%) |
| A952 | R1 diff. | USB | 434 | 131 (28.4%) | 330 (71.6%) |
| A953 | R1 undiff. ESC | Ab_anti-CDH1 | 342 | 347 (93.0%) | 26 (7.0%) |
| A954 | R1 diff. | Ab_anti-CDH1 | 217 | 106 (43.8%) | 136 (562%) |
| A955 | EB3 undiff. ESC | USB | 252 | 234 (81.3%) | 54 (18.7%) |
| A436 | EB3 diff. | USB | 505 | 141 (23.3%) | 463 (76.7%) |

Data obtained from the undifferentiated and differentiated states of R1 ES cells labeled by either the USB method or the Ab method are shown (Fig. 4). The cells were classified into 11 clusters using the uniform manifold approximation and projection (UMAP) method.

Clusters 0, 1, and 3 were considered to correspond to pluripotent cell, naive cells, and primed pluripotent cells, respectively, and to be cells in the undifferentiated state, from the expressed genes (Fig. 4, and Table 4).

**[Table 4]**

| Cluster ID | Top 20 of up-regulated genes | Cell types | E-Cad + or - |
|---|---|---|---|
| 0 | Pim2, Dnmt3b, Car2, Gm19792, Gng3, Pou3f1, Olfr1388, Cd59b, 2810429104Rik, Pou5f1, Wnt8a, Tdgf1, Hmga1, Snrpn, Dut, L1td1, Hspd1, Olfr1459, Psat1, Trh | Pluripotent cells | + |
| 1 | Dppa5a, Zfp42, Fbxo15, Tdh, Zfp600, Mybl2, Dnmt3l, Chchd10, Gm47654, Klf2, Zfp990, Ppcdc, Rps4l, Mkrn1, Hspb1, Gsta4, Sgk1, Platr3, Rhox5, Mycn | **Naïve ESCs** | + |
| 2 | Cdh11, Col3a1, Igf2, Lgals1, Gm49394, Fstl1, Igfbp4, Col10a1, Hmga2, Peg3, Col1a2, Dlk1, Postn, Itm2a, Mest, Acta2, H19, Ptn, Plagl1, Tagln | Vascular smooth muscle cells | - |
| 3 | Flt1, Cyp26a1, Krt8, Krtl8, PodxI, Car4, Rbp4, Spink1, Lhx1, Cldn6, Apoa1, Fam107b, Emb, Amot, Gpx3, Lefty1, Afp, Clu, Trh, Lefty2 | Primed pluripotent stem cells | + |
| 4 | IIdr2, Shh, Nr2f1, Sox21, Vezf1, Fabp7, Sox11, Lmx1a, Ckb, Fzd3, H1f0, Slit2, Tcf12, Ntn1, Spon1, Nnat, Jam2, Zic1, Sulf1, Rgs2 | Dopamine neurons | - |
| 5 | Tacstd2, Krt15, Krt6a, Perp, Sfn, F3, Anxa1, Wfdc2, Dsp, Krt19, Anxa2, Dsc2, Ccnd2, Arl4c, Igfbp5, Krt8, Krt17, Krt14, Bcl11b, Pitx1 | Epithelial cells | + |
| 6 | Crabp1, Ly6a, Ccl7, S100a4, Lrrc15, Ccl2, Fbln2, Bgn, Thy1, Lox, Dcn, Timp1, Tnc, S100a6, Gsto1, Emp1, Ctsl, Spp1, Mmp3, AC160336.1 | MEFs (feeder cells) | - |
| 7 | Dcx, Stmn4, Nhlh2, Gpm6a, Chgb, Map2, Kif5c, Cdk5r1, Tubb3, Tuba1a, Basp1, Map1b, Tubb2b, Sox11, Gap43, Ina, Nnat, Nefm, Fnbp1l, Mcf2l | Neural cells | - |
| 8 | Evi2a, Fcer1g, C3ar1, Tyrobp, C1qc, C1qb, Mpeg1, C1qa, Nfam1,Laptm5, Lyz2, Ptprc, Lpl, Lgmn, Lgals3, Ctsb, Adcy7, Cxcl16, Apoe, Spp1 | Macrophages | - |
| 9 | Gpr17, Cdh19, Ngfr, Moxd1, Plp1, Mef2c, Cdh6, Kctd12, Timp3, Zeb2, Pls3, Postn, Prss23, Serpine2, Lima1, Gap43, Nefl, Cryab, Nefm, Dnajc1 | Glial cells | - |
| 10 | Cdh5, Esam, Cldn5, Ccm2l, Kdr, Hapln1, Rasip1, Plxnd1, Esm1, Mmrn2, Cd34, Cd93, Tspanl8, Egfl7, Dok4, Flt1, Hdac7, Pf4, Mest, Irf2 | Vascular endothelial cells | - |

It is worth noting that cells in these clusters labeled by two different methods fell into the same clusters (Fig. 9). In other words, the expression profile of the cells labeled by the USB method of the present invention highly resembled the expression profile of the cells labeled by the conventional Ab method. Accordingly, it was revealed that the impact of the experimental operations by the USB method was negligible not only on cell growth but also on the gene expression profile of each cell.

Clusters other than 0, 1, and 3 such as vascular smooth muscle cells (cluster 2), dopamine neurons (cluster 4), epithelial cells (cluster 5), and neural cells (cluster 7) were identified as differentiated cells, and cell clusters thought of as macrophages (cluster 8), glial cells (cluster 9), and vascular endothelial cells (cluster 10) were detected. Mouse embryonic fibroblast (MEF) feeder cells which was not able to completely be removed were detected as cluster 6.

The cells labeled by the USB method were revealed to be present in all the clusters from the analysis of the cell samples belonging to each cluster (Figs. 4 and 9), indicating that different types of differentiated cells can indeed be trapped by this method.

In the differentiated cell clusters 2, 4, and 7, the numbers of cells labeled by the Ab method were reduced compared to the cells labeled by the UBS method (Figs. 4 and 9). This is considered that the expression of Cdh1 genes were suppressed in these differentiated cells, leading to a decrease in labeling efficiency by the Ab method using the CDH1 antibody. In Cdh1-positive clusters (clusters 0, 1, 3, and 5), almost the same numbers of cells were detected between the samples labeled by the USB method and the Ab method. However, in Cdh1-negative clusters (clusters 2, 4, 6, 7, 8, 9, and 10), the numbers of cells labeled by the Ab method were clearly lower than those by the USB method (Fig. 5), indicating the limitation of the Ab method for detecting differentiated cells.

**[Table 5]**

| Tag ID | Cells | Labeling methods | Total cell no. | E-Cad(+) cell no. (Clusters 0, 1, 3 and 5) | E-Cad(-) cell no. (Clusters 2, 4, 6, 7, 8, 9 and 10) |
|---|---|---|---|---|---|
| A951 | R1 undiff. ESC | NHS-Biotin labeling | 347 | 310 (85.9%) | 51 (14.1%) |
| A952 | R1 diff. ESC | NHS-Biotin labeling | 446 | 131 (28.4%) | 330 (71.6%) |
| A953 | R1 undiff. ESC | Biotin-anti-E-Cadherin labeling | 345 | 347 (93.0%) | 26 (7.0%) |
| A954 | R1 diff. ESC | Biotin-anti-E-Cadherin labeling | 222 | 107 (44.2%) | 135 (55.8%) |

This trend was observed in the EB3 line, and the USB method was shown to be capable of applying to different ES cell lines (Fig. 10).

CDH1-negative cells are thought not to have been labeled by the anti-CDH1 antibody, and should not be detected. Nevertheless, in the antibody-treated cells, the Cdh1-negative cells were clearly included, albeit in a clearly reduced number (Table 5, Figs. 4 and 5). As potential, non-specific binding of antibodies or streptavidin to cells is thought to be the cause. In fact, the present inventors have confirmed that barcoded oligo DNA is amplified in both streptavidin alone and isotype control antibody/streptavidin treated samples (Figs. 13 and 14). Since the barcoded oligo DNA was not amplified in the samples without labeling, the amplification observed in streptavidin alone and isotype control antibody treated samples (Fig. 14, arrows) is likely to be caused by non-specific binding of streptavidin and/or isotype control antibody to cells.

Since the amplification by scRNA-seq is thought to be more sensitive than that by flow cytometry, some of the cells that express no CDH1 (or express very tiny amount) may be classified as positive cells, due to the non-specific binding of streptavidin and/or isotype control antibody. However, in cell labeling for multiplexing scRNA-seq, it is very important for all cells in the samples to be evenly labeled, and the tiny amount of non-specific binding by the Ab method itself is not considered to impact on the results of multiplexing.

### EXAMPLE 4 Sulfo-NHS-Esterification of Barcoded Oligo DNA

In this EXAMPLE, sulfo-NHS-esterification of barcoded oligo DNA was performed. The materials used are as follows.
· Oligo DNA with -COOH modified at the 5' end;
   For a verification experiment for cell labeling, 6-FAM modification at the 3' end was also performed. (6-FAM modification is not required for sample multiplexing of scRNA-seq.)
· EDC (1-ethyl-3-[3-dimethylaminopropyl]carbodiimide hydrochloride) (Thermo Fisher SCIENTIFIC (TM)), A35391 (10 × 1 mg, No-Weigh (TM) Format);
· Sulfo-NHS (Thermo Fisher SCIENTIFIC (TM)), A39269 (10 × 2 mg, No-Weigh (TM) Format);
· Buffer solution for activation (0.1 M MES, 0.5 M NaCl, pH 6.0);
· PD MidiTrap G-25 (Cytiva, Inc., 28918008);
   and
· Nanosep centrifugal filter device, fractionated molecular weight of 3K (NIPPON Genetics Co., Ltd., OD003C33)

The methods used in the EXAMPLE are as follows.
(4-i) Load 918 µL buffer solution for activation into a reaction tube;
(4-ii) Dissolve 1 mg EDC in 100 µL buffer solution for activation and add 40 µL thereof to the reaction tube;
(4-iii) Add 2 nmol (20 µL) oligo DNA to the reaction tube;
(4-iv) Dissolve 2 mg sulfo-NHS in 40 µL buffer solution for activation and add 22 µL thereof to the reaction tube;
(4-v) Allow to stand for 15 minutes at room temperature;
   (the operations afterwards are performed at 4°C or on ice)
(4-vi) Remove unreacted reagents using PD MidiTrap G-25;
(4-vii) Substitute the buffer solution for PBS by using the Nanosep centrifugal filter device (buffer substitution by ultrafiltration). This operation is repeated twice to concentrate sulfo-NHS-esterified oligo DNA (NEO).

The sulfo-NHS-esterified oligo DNA obtained above was stored at -80°C until used for subsequent examples.

### EXAMPLE 5 Verification of Cell Labeling Efficiency by Sulfo-NHS-Esterified Oligo DNA (NEO)

In this EXAMPLE, cell were labeled using sulfo-NHS-esterified oligo DNA (NEO) (single-step method) and the labeling efficiency was verified.

All operations and reaction of this EXAMPLE were performed at 4°C or on ice.
(5-i) Prepare 1 × 10⁶ cells dispersed in single cells, according to the method in "3. Preparation of SINGLE CELLs for scRNA-seq Analysis" in "Materials and Methods".
(5-ii) Dilute 4 ng sulfo-NHS-esterified oligo DNA (NEO) with 20 µL of 0.1% BSA/PBS.
(5-iii) Suspend the dispersed single cells prepared in (5-i) in NEO solution prepared in (5-ii) to treat for 20 minutes on ice.
(5-iv) Add 3 mL of 3% FBS/PBS thereto to centrifuge (300x g, 10 minutes).
(5-v) Remove supernatant and suspend to 1 mL Cell Staining Buffer (Catalog# 420201) (BioLegend, Inc.) to centrifuge (300x g, 10 minutes). This operation is repeated three times.

The above labeled cells were used for SINGLE CELL RNA-seq analysis (scRNA-seq analysis).

Further, the labeling efficiency of cells was verified as follows.

The suspension solution of the cells labeled by using 6-FAM modified, sulfo-NHS-esterified oligo DNA (NEO) was passed through a Cell strainer, and then flow cytometry analysis was performed.

The results are shown in Fig. 15. Compared to the table labeling control (0.01% labeling rate), it was confirmed that almost all cells (about 99.6%) were sufficiently labeled in the single-step method labeling in this EXAMPLE.

### EXAMPLE 6 Multiplexed scRNA-seq Analysis of Various Cells Labeled by Single-Step Method

Undifferentiated ES cells cultured in naive pluripotent stem cell maintenance medium (2i medium), undifferentiated ES cells cultured in normal ES cell medium (normal ESC medium), differentiated ES cells cultured in LIF(-) serum medium for 5 days (Day 5 differentiated), and differentiated ES cells cultured in LIF(-) serum medium for 10 days (Day 10 differentiated) were each labeled with different barcode tags by the single-step method, and then multiplexed scRNA-seq analysis was performed (Fig. 16).

9 cell types were detected by clustering with UMAP. Cell types and marker genes (top representative 20) of the 9 cell types are as follows.

**[Table 6]**

| Table 6 Top 20 of identified marker genes with the UMAP cluster list | | |
|---|---|---|
| Cluster ID | Top 20 of identified marker genes | Cell types |
| 0 | Tdh, Hspb1, Rps4l, Mylpf, Zfp42Klf2, Ckb, Zfp600, Dnmt3l, Hsd17b14, Gsta4, Gpx1, Dppa5a, Rpt10l, Stmn2, L1td1, Ccnel, Utf1, Trim28, Rhox5 | Naïve pluripotent cells |
| 1 | Handl, Mest, Prtg, Tmem88, Pmp22, Hmga2, Bmp4, Peg3, Igfbp4, Csrp2, Tgfb2, Gpc3, Maged1, Nrp1, Stard8, Capn6, Dok4, Gpx3, Idf2r, Grb10 | Cardiomyocytes |
| 2 | Leftyl, Car2, Pim2, Dnmt3b, Cer1, Fgf5, Fgf8, Gsc, Hmga1, Emb, Pycr2, Cyp26a1, T, Trh, Pkdcc, Igfbp3, Lhx1, Otx2, Lefty2, Flt1 | Primed pluripotent stem cells |
| 3 | Dppa5a, Chchd10, Ldhb, Sox2, Mkrn1, Mybl2, Esrrb, Mt2, Alpl, Utf1, Asns, Ifitm1, Zfp42, Gm47654, Olfr46, Ppcdc, Gm42669, Txnip, Mt1, Gm47031 | Intermediate pluripotent cells |
| 4 | Acta2, Col3a1, Ptn, Tagln, Igfbp7, Col1a1, Col1a2, Dlk1, Plagl1, Myl9, Itm2a, Fstl1, Sparc, Lgals1, Igr2, Gm49394, Actg2, Loxl2, Bgn, H19 | Vascular smooth muscle cells |
| 5 | Krt7, Krt18, Krt19, Krt8, Cryab, Anxa1, Dusp9, Sfn, Peg10, Anxa2, Igfbp2, Tinagl1, Bex1, S100a6, Slc2a1, Pdlim1, Crip1, Lgals3, Fabp3, H19 | Epithelial cells |
| 6 | Ttr, Rbp4, Apoal, S100g, Ctsh, Apob, Spink1, Apom, Cited1, Dab2, Lgmn, Ctsl, Podxl, Apoe, Col4a1, Emb, Col4a2, Gpx3, Car4, Lama1 | Visceral endoderm cells |
| 7 | Crabpl, S100a4, Dcn, Lox, Thy1, Ly6a, Col12a1, Bgn, Tnc, Fbln2, Timp2, Gsto1, Timp1, Rps18-ps6, Col1a2, S100a6, Ifitm3, Lgals1, AC160336.1, Col1a1 | MEFs (feeder cells) |
| 8 | Tyrobp, C1qc, C1qb, Fcrls, Ccl4, F13a1, Fcer1g, C1qa, Mrc1, Cx3cr1, Lyz2, Kdr, Csf1r, Fxyd5, Coro1a, Selenop, Egfl7, Laptm5, Ctsb, Ctsd | Macrophages |

The results are shown in Fig. 17 and Table 7.

**[Table 7]**

| Table 7 The number of cells that belong to each UMAP cluster | | | | | |
|---|---|---|---|---|---|
| Cluster ID | 2i medium (A952) | normal ESC medium (A953) | Day 5 differentiated (A954) | Day 10 differentiated (A955) | Total |
| 0. Naive pluripotent cells | 1475 | 308 | 25 | 24 | 1832 |
| 1. Cardiovascular cells | 239 | 67 | 1110 | 86 | 1502 |
| 2. Primed pluripotent cells | 568 | 535 | 58 | 52 | 1213 |
| 3. Intermediate pluripotent cells | 639 | 142 | 156 | 181 | 1118 |
| 4. Vascular smooth muscle cells | 173 | 33 | 83 | 817 | 1106 |
| 5. Epitherial cells | 114 | 22 | 422 | 116 | 674 |
| 6. Visceral endoderm cells | 83 | 23 | 88 | 139 | 333 |
| 7. MEF | 58 | 68 | 2 | 1 | 129 |
| 8. Macrophage | 10 | 5 | 29 | 71 | 115 |
| Total | 3359 | 1203 | 1973 | 1487 | 8022 |

In the 2i medium, many naive pluripotent cells were confirmed, and in the normal ESC medium, primed pluripotent cells were the most. In Day 5 differentiated, cardiovascular cells accounted for the majority, and in Day 10 differentiated, differentiated cells were detected, including mainly vascular smooth muscle cells.

SEQUENCE LISTING

## Claims

1. A method for labeling a cell with a barcode, comprising:
(1) for a cell group containing a plurality of single cells, directly or indirectly bringing a cell surface protein of the cell into contact with a modified barcode; or
(2) directly or indirectly bringing a cell surface protein of a single cell into contact with a modified barcode.

2. A method for labeling a cell with a barcode, comprising:
(ia) for a cell group containing a plurality of single cells, biotinylating cell surface proteins of the cell;
(ib) biotinylating cell surface proteins of a single cell; or
(ic) biotinylating cell surface proteins of a plurality of cells followed by separating the biotinylated cells into cell groups containing a plurality of single cells, or into a single cell,
and
(ii) bringing each cell group or each single cell into contact with a barcoded biotin binding substance.

3. The method according to claim 2, wherein the method comprises:
(ia) for the cell group containing a plurality of single cells, biotinylating the cell surface protein;
and
(ii) bringing each cell group into contact with the barcoded biotin binding substance.

4. The method according to claim 2 or 3, biotinylating an amino group, a sulfhydryl group, or a carboxyl group of the cell surface protein in (i).

5. The method according to any one of claims 2 to 4, wherein a reagent used for biotinylation in (i) is selected from the group consisting of sulfo-N-hydroxysuccinimide-biotin, N-hydroxysuccinimide-biotin, and pentafluorophenyl-biotin,
wherein the binding of biotin with sulfo-N-hydroxysuccinimide, N-hydroxysuccinimide, or pentafluorophenyl can comprise a spacer.

6. The method according to claim 5, wherein the reagent used for biotinylation in (i) has been cryopreserved.

7. The method according to any one of claims 2 to 6, wherein the biotin binding substance is selected from the group consisting of streptavidin, avidin, and anti-biotin antibody.

8. The method according to claim 7, wherein the biotin binding substance is streptavidin.

9. The method according to any one of claims 1 to 8, wherein the barcode is oligo DNA.

10. The method according to claims 1 to 9, wherein the method is for labeling a viable cell.

11. The method according to any one of claims 1 to 9, wherein the method is for labeling a fixed cell.

12. A kit for use in a method for labeling a cell with a barcode, comprising a reagent for biotinylating a cell surface protein of the cell, and
a barcoded biotin binding substance.

13. The kit according to claim 12, wherein the reagent for biotinylating the cell surface protein of the cell is sulfo-N-hydroxysuccinimide-biotin.

14. A method for multiplexed analysis of a cell sample, comprising:
(ia) for a cell group containing a plurality of single cells, biotinylating cell surface proteins of the cell;
(ib) biotinylating cell surface proteins of a single cell; or
(ic) biotinylating cell surface proteins of a plurality of cells followed by separating the biotinylated cells into cell groups containing a plurality of single cells, or into a single cell,
(ii) bringing each cell group or each single cell into contact with a barcoded biotin binding substance,
(iii) mixing all or a part of the cell groups or single cells barcode-labeled in (ii), and
(iv) analyzing the mixture of the cells of (iii).

15. The method according to claim 14, comprising: (ia) for the cell group containing a plurality of single cells, biotinylating the cell surface protein;
and
(ii) bringing each cell group into contact with the barcoded biotin binding substance.

16. The method according to claim 15, wherein the analysis is cellular RNA analysis.

17. A method for labeling a cell with a barcode, comprising:
(1) bringing a cell group containing a plurality of single cells into contact with a barcode modified with a compound that binds to an amino group of a cell surface protein of the cell; or
(2) bringing a single cell into contact with a barcode modified with a compound that binds to an amino group of the cell surface protein of the cell.

18. The method according to claim 17, wherein the compound that binds to the amino group of the cell surface protein of the cell is selected from the group consisting of sulfo-N-hydroxysuccinimide, N-hydroxysuccinimide, and pentafluorophenyl.

19. The method according to claim 18, wherein the compound that binds to the amino group of the cell surface protein of the cell has been cryopreserved.

20. The method according to any one of claims 17 to 19, wherein the barcode is oligo DNA.

21. The method according to claims 17 to 20, wherein the method is for labeling a viable cell.

22. The method according to any one of claims 17 to 21, wherein the method is for labeling a fixed cell.

23. A kit for use in a method for labeling a cell with a barcode, comprising a barcode modified with a compound that binds to an amino group of a cell surface protein of the cell.

24. The kit according to claim 23, wherein the compound that binds to the amino group of the cell surface protein of the cell is sulfo-N-hydroxysuccinimide.

25. A method for multiplexed analysis of a cell sample, comprising:
(1A) bringing a cell group containing a plurality of single cells into contact with a barcode modified with a compound that binds to an amino group of a cell surface protein of the cell; or
(1B) bringing a single cell into contact with a barcode modified with a compound that binds to an amino group of a cell surface protein of the cell,
(2) mixing all or a part of the cell groups or single cells barcode-labeled in (1), and
(3) analyzing the mixture of the cells of (2).

26. The method according to claim 25, wherein the analysis is cellular RNA analysis.
